# FASCICULE DE BREVET EUROPEEN

(11) **EP 0 699 187 B1**
(45) Date de publication et mention de la délivrance du brevet: **22.01.1997**
(21) Numéro de dépôt: 94916294.5
(22) Date de dépôt: 17.05.1994
(51) Int. Cl.: C07D 211/26, C07D 211/34, A61K 31/445

(54) **DERIVES DE BETA,BETA-DIMETHYL-4-PIPERIDINEETHANAMINE COMME INHIBITEURS DE LA BIOSYNTHESE DU CHOLESTEROL**
BETA,BETA-DIMETHYL-4-PIPERIDINETHANAMIN-DERIVATE ALS INHIBITOREN DER CHOLESTERIN-BIOSYNTHESE
BETA,BETA-DIMETHYL-4-PIPERIDINEETHANAMINE DERIVATIVES AS INHIBITORS OF THE CHOLESTEROL BIOSYNTHESIS

(30) Priorité: 17.05.1993 FR 9305915
(43) Date de publication de la demande: 06.03.1996
(73) Titulaire: FOURNIER INDUSTRIE ET SANTE, F-75008 Paris (FR)
(72) Inventeur: BINET, Jean, F-21121 Fontaine-Les-Dijon (FR); SAMRETH, Soth, F-21600 Longvic (FR); DE FORNEL, Daniel, F-21000 Dijon (FR)
(74) Mandataire: Clisci, Serge
(86) Numéro de dépôt international: FR9400584
(87) Numéro de publication internationale: WO9426713

(56) Documents cités:
- EP-A- 0 420 116
- EP-A- 0 468 457
- FR-A- 2 300 552
- GB-A- 991 509
- US-A- 4 248 877

## Description

### Domaine de l'invention

La présente invention concerne, en tant que produits industriels nouveaux, des dérivés de β,β-diméthyl-4-pipéridineéthanamine, inhibiteurs de la biosynthèse du cholestérol, notamment de l'époxysqualène cyclase, chez les mammifères et les champignons, leur procédé de préparation et leur utilisation en thérapeutique en tant qu'agents hypocholestérolémiants, hypolipémiants, antiathéromateux et antifongiques.

### Art antérieur

De nombreuses études ont démontré (i) le lien existant entre un taux de cholestérol élevé et les risques cardiovasculaires associés, et (ii) l'intérêt de normaliser ledit taux de cholestérol.

Dans ce but, on sait que l'on a déjà développé un certain nombre de composés inhibiteurs de la biosynthèse du cholestérol comme par exemple les inhibiteurs de l'HGM-CoA réductase.

Il apparaît préférable d'inhiber cette biosynthèse dans son stade final, dès lors que les premiers précurseurs peuvent intervenir dans la synthèse d'autres molécules biologiques importantes.

On a donc cherché à développer des inhibiteurs de l'époxysqualène cyclase et de la squalène époxydase, qui sont les enzymes qui catalysent la transformation du squalène, via le 2,3-oxydosqualène, en lanostérol qui est le premier composé de la famille des stérols à être formé.

De la même façon, la biosynthèse d'ergostérol endogène est nécessaire à la croissance et à la reproduction de nombreux champignons. Des composés inhibant cette biosynthèse et notamment les deux enzymes précédents présentent donc des propriétés anti-fongiques très intéressantes.

On connaît déjà un certain nombre de composés inhibiteurs de l'époxysqualène cyclase utiles en tant qu'agents inhibiteurs de la biosynthèse du cholestérol.

Par exemple la demande internationale WO-A-89/08450 et le brevet US-A-5084461 décrivent des dérivés de décalines et d'azadé-calines et la demande de brevet EP-A-468434 décrit des éthers ou thioéthers de 4-hydroxypipéridine. Les demandes de brevets EP-A-468457 et EP-A-420116 décrivent des dérivés de β-méthyl-4-pipéridineéthanol et des composés alkyl-4-pipéridinol en tant qu'inhibiteurs de la squalène époxydase et signalent leur utilité en tant qu'agents antiathéromateux et antifongiques.

Aucun de ces documents antérieurs ne décrit ni ne suggère (i) des dérivés de β,β-diméthyl-4-pipéridineéthanamine en tant que produits, et (ii) leur utilisation comme inhibiteurs de la biosynthèse du cholestérol et notamment de l'époxysqualène cyclase.

### Objet de l'invention

La présente invention propose donc des dérivés de β,β-diméthyl-4-pipéridineéthanamine, inhibiteurs de la biosynthèse du cholestérol et notamment de l'époxysqualène cyclase.

La présente invention se rapporte aux composés choisis parmi l'ensemble constitué par les β,β-diméthyl-4-pipéridineéthanamine de formule : 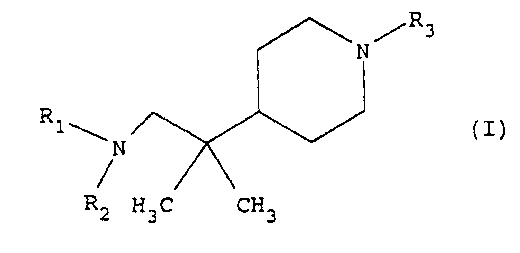 dans laquelle
- R₁ et R₂, identiques ou différents, représentent chacun l'atome d'hydrogène, un groupe alkyle en C₁-C₄, un groupe acyle en C₂-C₅, un groupe phénylméthyle ou forment avec l'atome d'azote auquel ils sont liés un groupe pipéridinyle,
- R₃ représente
   - un groupe alkyle en C₁-C₁₂ linéaire ou ramifié, éventuellement substitué par :
      a) un groupe imidazolyle ou
      b) un groupe phényle lui-même éventuellement substitué par un groupe alkyle linéaire ou ramifié en C₁-C₄,
   - un groupe alkyle linéaire ou ramifié en C₃-C₁₀ comportant une ou plusieurs liaisons C=C ou C≡C, et éventuellement substitué par un groupe phényle,
   - un groupe alkyloxy en C₁-C₄, linéaire ou ramifié, substitué par un groupe para-chlorophényle,
   - un groupe -CO-R₈, dans lequel R₈ représente un groupe alkyle linéaire ou ramifié en C₈-C₁₁ éventuellement substitué par un groupe imidazolyle, un groupe alkyloxy en C₁-C₄, linéaire ou ramifié, substitué par un groupe para-chlorophényle ou un groupe alkyle en C₂-C₄, linéaire ou ramifié, comportant au moins une liaison double et substitué par un groupe phényle,
   - un groupe alkyle linéaire ou ramifié en C₁-C₄ substitué par l'un des groupes suivants :
      un groupe -COOR₄, dans lequel R₄ représente soit un atome d'hydrogène soit un groupe alkyle linéaire ou ramifié en C₁-C₄,
      un groupe 1H-isoindole-1,3(2H)-dione,
      un groupe NR₅R₆, dans lequel R₅ et R₆, identiques ou différents, représentent chacun un atome d'hydrogène ou un groupe alkyle linéaire ou ramifié en C₁-C₄,
      un groupe -NH-CO-R₇ ou un groupe -CO-NH-R₇ dans lesquels R₇ représente un groupe alkyle linéaire ou ramifié en C₁-C₆ ; et,
leurs sels d'addition.

L'invention concerne également un procédé de préparation des composés de formule I qui est décrit ci-après.

### Description détaillée de l'invention

Les groupes alkyle préférés sont les groupes méthyle, éthyle, n-propyle, isopropyle, n-butyle, n-pentyle, tertiobutyle, 2-méthylpropyle, 5-méthylpentyle, octyle, nonyle, décyle, undécyle et dodécyle.

Les groupes alkyle en C₃-C₁₀ comportant une ou plusieurs liaisons C=C ou C≡C préférés sont les groupes 2-éthèn-1-yle, 4-méthyl-2-butèn-1-yle et 6,6-diméthyl-hept-2-èn-4-yn-1-yle.

Par sels d'addition on vise ici les sels d'addition d'acide et les sels d'ammonium.

Par sels d'addition d'acide on entend les sels obtenus avec les acides organiques comme par exemple les acides 4-mèthylbenzènesulfonique, (E)-2-butènedioïque, (Z)-2-butènedioïque, éthanedioïque, méthane-sulfonique, paratoluènesulfonique, acétique, citrique, aspartique, glutamique, succinique, propionique ou avec les acides minéraux, comme par exemple les acides chlorhydrique, bromhydrique, sulfurique, nitrique et phosphorique.

Par sels d'ammonium on entend les sels d'ammonium obtenus par réaction d'un halogénure hydrocarboné notamment en C₁-C₁₄, en particulier un halogénure d'alkyle, comme par exemple l'iodure de méthyle, avec un composé de formule I sous forme de base.

Les composés de formule (I) selon l'invention peuvent être préparés suivant un procédé caractérisé en ce qu'il comprend les étapes consistant à :
(i) soumettre à une réaction de N-alkylation ou N-acylation un composé de formule : 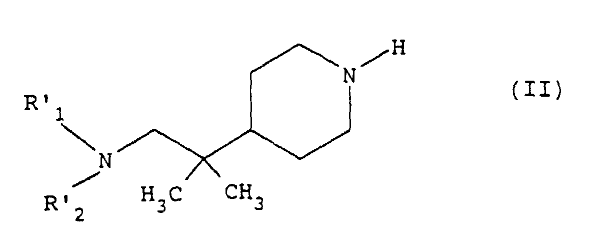 dans laquelle :
   R'₁ et R'₂, identiques ou différents, représentent chacun un atome d'hydrogène, un groupe alkyle en C₁-C₄, un groupe phénylméthyle ou forment avec l'atome d'azote auquel ils sont liés un groupe pipéridinyle, par réaction avec un composé de formule R'₃-X où X représente un halogène comme par exemple un atome de brome ou de chlore et R'₃ représente :
   un groupe alkyle en C₁-C₁₂ linéaire ou ramifié éventuellement substitué par un groupe imidazolyle ou un groupe phényle lui-même éventuellement substitué par un groupe alkyle linéaire ou ramifié en C₁-C₄,
   un groupe alkyle linéaire ou ramifié en C₃-C₁₀ comportant une ou plusieurs liaisons C=C ou C≡C,
   un groupe -CO-R'₈ dans lequel R'₈ représente un groupe alkyle linéaire ou ramifié en C₈-C₁₁ éventuellement substitué par un groupe imidazolyle,
   un groupe alkyle linéaire ou ramifié en C₁-C₄ substitué par un groupe -COOR'₄ dans lequel R'₄ représente un atome d'hydrogène ou un groupe alkyle linéaire ou ramifié en C₁-C₄,
   un groupe 1H-isoindole-1,3(2H)-dione,
   en présence ou non d'un solvant polaire ou non polaire et aprotique, comme par exemple l'acétonitrile, le trichlorométhane ou le N,N-diméthylformamide, en présence ou non d'un sel de métal alcalin comme par exemple le carbonate de potassium, l'iodure de potassium ou l'iodure de sodium et en présence ou non d'une base forte, comme par exemple la N,N-diéthyléthanamine, notamment dans le cas d'une réaction de N-acylation, à raison de 1 mole de composé de formule Il pour 1,1 moles de composé de formule R'₃-X, à une température comprise entre 0°C et 200°C et pendant au moins 1 heure (i.e. une heure à plusieurs jours) pour obtenir un composé de formule : 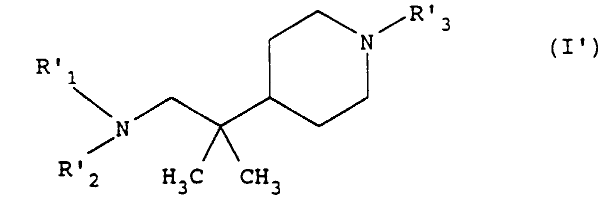 dans laquelle R'₁ , R'₂ et R'₃ sont définis comme ci-dessus;
(ii) si nécessaire, soumettre les composés de formule I' ainsi obtenus à au moins un des traitements suivants :
   (a) les composés de formule I' dans laquelle R'₃ représente un groupe alkyle linéaire ou ramifié en C₃-C₁₀ comportant une ou plusieurs liaisons C=C ou C≡C et R'₁ et R'₂ sont définis comme précédemment, sont transformés selon les méthodes connues de l'homme de l'art, notamment par hydrogénation catalytique dans un appareil de Parr, dans un alcool, comme par exemple le méthanol, en présence d'un catalvseur tel le charbon palladié, en composés de formule I dans laquelle R₁ et R₂, identiques ou différents, représentent chacun un atome d'hydrogène, un groupe alkyle linéaire ou ramifié en C₁-C₄, un groupe phénylméthyle ou forment avec l'atome d'azote auquel ils sont liés un groupe pipéridinyle et R₃ représente une chaîne alkyle en C₃-C₁₀ linéaire ou ramifié;
   (b) on réalise une hydrolyse des composés de formule I' dans laquelle R'₁ et R'₂ sont définis comme précédemment et R'₃ représente un groupe alkyle linéaire ou ramifié en C₁-C₄ substitué par un groupe 1H-isoindole-1,3(2H)-dione, selon les méthodes connues de l'homme de l'art, notamment en présence d'hydrate d'hydrazine, éventuellement suivie d'une N-alkylation de l'amine primaire ainsi obtenue, par un groupe alkyle en C₁-C₄ approprié pour obtenir les composés de formule I dans laquelle R₁ et R₂, identiques ou différents, représentent chacun un atome d'hydrogène, un groupe alkyle linéaire ou ramifié en C₁-C₄, un groupe phénylméthyle ou forment avec l'atome d'azote auquel ils sont liés un groupe pipéridinyle et R₃ représente un groupe alkyle linéaire ou ramifié en C₁-C₄ substitué par un groupe NR₅R₆ dans lequel R₅ et R₆, identiques ou différents, représentent chacun un atome d'hydrogène ou un groupe alkyle linéaire ou ramifié en C₁-C₄;
   c) on réalise une N-acylation de l'amine primaire obtenue au stade (b) précédent selon les méthodes connues de l'homme de l'art, comme par exemple par réaction avec un halogènure d'acide de formule X-CO-R₇ dans laquelle X représente un atome d'halogène tel le chlore ou le brome et R₇ un groupe alkyle linéaire ou ramifié en C₁-C₆, pour obtenir les composés de formule I dans laquelle R₁ et R₂, identiques ou différents, représentent chacun un atome d'hydrogène, un groupe alkyle linéaire ou ramifié en C₁-C₄, un groupe phénylméthyle ou forment avec l'atome d'azote auquel ils sont liés un groupe pipéridinyle, et R₃ représente un groupe alkyle linéaire ou ramifié en C₁-C₄ substitué par un groupe -NH-CO-R₇ dans lequel R₇ représente un groupe alkyle linéaire ou ramifié en C₁-C₆ ;
   (d) on amidifie les composés de formule I' dans laquelle R'₁ et R'₂ sont définis comme précédemment et R'₃ représente un groupe alkyle linéaire ou ramifié en C₁-C₄ substitué par un groupe -COOR'₄, dans lequel R'₄ représente un atome d'hydrogène ou un groupe alkyle linéaire ou ramifié en C₁-C₄, par réaction avec une amine primaire appropriée, selon les méthodes connues de l'homme de l'art pour obtenir les composés de formule I dans laquelle R₁ et R₂, identiques ou différents, représentent chacun un atome d'hydrogène, un groupe alkyle linéaire ou ramifié en C₁-C₄, un groupe phénylméthyle ou forment avec l'atome d'azote auquel ils sont liés un groupe pipéridinyle et R₃ représente un groupe alkyle linéaire ou ramifié en C₁-C₄ substitué par un groupe -CO-NH-R₇ dans lequel R₇ représente un groupe alkyle linéaire ou ramifié en C₁-C₆;
   (e) on réalise la réduction des composés de formule I' dans laquelle R'₁ et R'₂ sont définis comme précédemment et R'₃ représente un groupe -CO-R'₈, dans lequel R'₈ représente un groupe alkyle linéaire ou ramifié en C₈-C₁₁ éventuellement substitué par un groupe imidazolyle, selon les méthodes connues de l'homme de l'art, en présence d'un agent réducteur, comme par exemple un dérivé d'hydrure d'aluminium, puis traite avec une base forte tel l'hydroxyde de sodium pour obtenir les composés de formule I dans laquelle R₁ et R₂, identiques ou différents, représentent chacun un atome d'hydrogène, un groupe alkyle en C₁-C₄, un groupe phénylméthyle ou forment avec l'atome d'azote auquel ils sont liés un groupe pipéridinyle et R₃ représente un groupe alkyle linéaire ou ramifié en C₉-C₁₂ éventuellement substitué par un groupe imidazolyle;
   (f) on acyle les composés de formule I' dans laquelle un des deux groupes R'₁ et R'₂ représente l'atome d'hydrogène, l'autre pouvant être un atome d'hydrogène, un groupe alkyle en C₁-C₄ ou un groupe phénylméthyle et R'₃ a la signification donnée à R₃, selon les méthodes connues de l'homme de l'art, notamment par réaction avec un anhydride d'acide, comme par exemple l'anhydride acétique pour obtenir les composés de formule I dans laquelle un des groupes R₁ ou R₂ représente un groupe acyle, l'autre pouvant être un atome d'hydrogène, un groupe alkyle en C₁-C₄ ou un groupe phénylméthyle et R₃ représente
      un groupe alkyle en C₁-C₁₂ linéaire ou ramifié éventuellement substitué par un groupe imidazolyle ou un groupe phényle lui-même éventuellement substitué par un groupe alkyle en C₁-C₄,
      un groupe alkyle linéaire ou ramifié en C₃-C₁₀ comportant une ou plusieurs liaisons C=C ou C≡C,
      un groupe -CO-R₈ dans lequel R₈ représente un groupe alkyle linéaire ou ramifié en C₈-C₁₁ éventuellement substitué par un groupe imidazolyle,
      un groupe alkyle linéaire ou ramifié en C₁-C₄ substitué par :
         un groupe -COOR₄ dans lequel R₄ représente un atome d'hydrogène ou un groupe alkyle linéaire ou ramifié en C₁-C₄,
         un groupe 1H-isoindole-1,3(2H)-dione,
         un groupe NR₅R₆ dans lequel R₅ et R₆, identiques ou différents, représentent chacun un atome d'hydrogène ou un groupe alkyle linéaire ou ramifié en C₁-C₄,
         un groupe -NH-CO-R₇ ou un groupe -CO-NH-R₇ dans lequel R₇ représente un groupe alkyle linéaire ou ramifié en C₁-C₆.

Par atome d'halogène on entend ici les atomes de fluor, de brome, de chlore et d'iode; les atomes d'halogène préférés sont le chlore et le brome, pour la synthèse, et l'iode, pour les sels d'ammonium.

Pour accéder aux composés de formule Il dans laquelle R'₁ et R'₂ sont définis comme précédemment on préconise de préparer par réaction de Mannich les dérivés de β,β-diméthyl-4-pyridineéthanamine de formule : 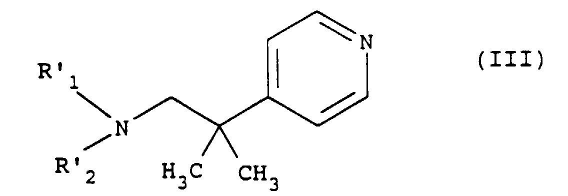 dans laquelle R'₁ et R'₂, identiques ou différents, représentent chacun un atome d'hydrogène, un groupe alkyle en C₁-C₄, un groupe phénylméthyle ou forment avec l'atome d'azote auquel ils sont liés un groupe pipéridinyle, selon les méthodes connues de l'homme de l'art, notamment par réaction de la (1-méthyléthyl)-4-pyridine, en solution dans l'acide acétique en présence de formaldéhyde, avec une amine appropriée, puis d'hydrogéner les composés de formule III ainsi obtenus, selon les méthodes connues de l'homme de l'art, notamment par hydrogénation catalytique dans un appareil de Parr, en solution dans un solvant comme par exemple l'acide acétique, en présence d'un catalyseur, comme par exemple le dioxyde de platine.

Les composés de formule Il et les composés intermédiaires de formule III, dans lesquelles R'₁ et R'₂ identiques ou différents représentent chacun un atome d'hydrogène, un groupe alkyle en C₁-C₄, un groupe phénylméthyle ou forment avec l'atome d'azote auquel ils sont liés un groupe pipéridinyle, sont des composés nouveaux.

Suivant un autre procédé selon l'invention, les composés de formule I', dans laquelle R'₁ et R'₂, identiques ou différents, représentent chacun un atome d'hydrogène, un groupe alkyle en C₁-C₄, un groupe phénylméthyle ou forment avec l'atome d'azote auquel ils sont liés un groupe pipéridinyle et R'₃ représente un groupe alkyle en C₁-C₁₂ linéaire ou ramifié ou un groupe alkyle linéaire ou ramifié en C₃-C₁₀ comportant une ou plusieurs liaisons C=C ou C≡C sont obtenus, selon les méthodes connues de l'homme de l'art, par réduction des dérivés de α,α-diméthyl-4-pipéridineacétamide correspondants de formule : 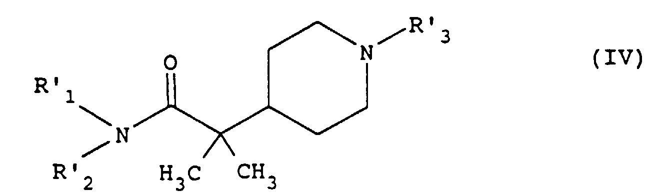 notamment par agitation durant une à trois heures à la température ambiante (15°-25°C) d'une solution dans un solvant approprié, comme par exemple le méthylbenzène, en présence d'un agent réducteur, comme par exemple l'hydrure de bis(2-méthoxyéthoxy)aluminium-sodium, à raison de une mole de a,a-diméthyl-4-pipéridineacétamide pour 0,4 à 0,5 mole d'agent réducteur, et chauffage à la température de reflux du mélange durant une à plusieurs heures.

Pour accéder aux composés de formule IV on préconise dans une première étape de soumettre un dérivé de 4-pipéridone de formule 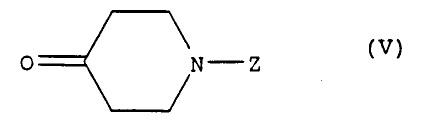 dans laquelle Z représente un groupe protecteur, comme par exemple les groupes phénylméthyle, 4-méthoxyphénylméthyle ou COOY dans lequel Y représente un groupe alkyle en C₁-C₄ ou un groupe phénylméthyle, avec un dérivé de formule (CH₃)₂-CH-CO₂-W dans laquelle W représente un groupe alkyle en C₁-C₄, selon les méthodes connues de l'homme de l'art, à une réaction d'aldolisation, notamment par réaction dans un solvant anhydre et aprotique, comme par exemple le tétrahydrofuranne, à la température de -70°C, pour obtenir un composé de formule : 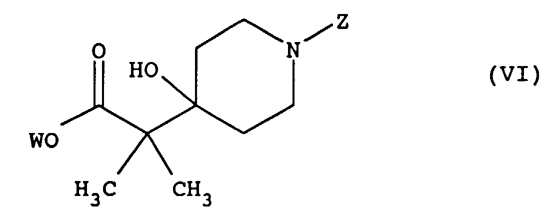 dans laquelle Z et W sont définis comme ci-dessus, puis on déshydrate le composé de formule VI selon les méthodes connues de l'homme de l'art, notamment par réaction avec le chlorure de thionyle dans un solvant halogéné et on traite par une base forte, comme par exemple l'hydroxyde de sodium, pour former un dérivé de l'acide α,α-diméthyl-1,2,3,6-tétrahydro-4-pyridine-acétique correspondant de formule :

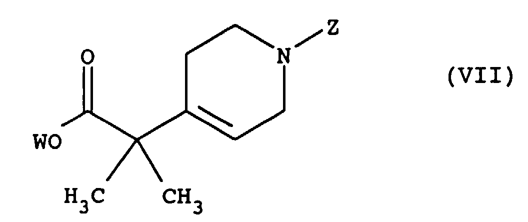

Les composés de formule VII sont ensuite déprotégés et hydrogénés, selon les méthodes connues de l'homme de l'art, notamment par hydrogénation dans un appareil de Parr, en solution dans un alcool, comme par exemple l'éthanol, en présence d'un catalyseur, comme par exemple le charbon palladié, pour conduire aux dérivés de formule 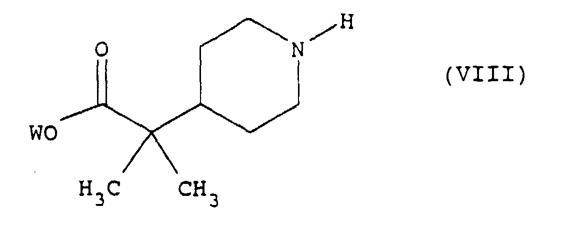 dans laquelle W représente un groupe alkyle en C₁-C₄.

Le dérivé de pipéridine de formule VIII ainsi obtenu est ensuite N-alkylé, suivant les méthodes connues de l'homme de l'art, notamment par réaction dans un solvant polaire, comme par exemple l'acétonitrile, avec un dérivé halogéné approprié, comme par exemple un dérivé bromé, pour obtenir un composé de formule : 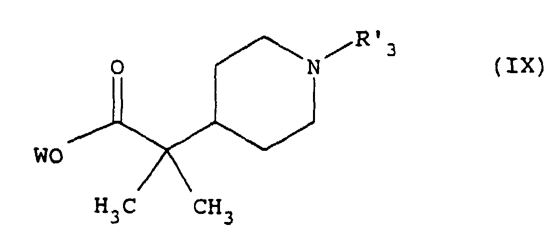 dans laquelle W est défini comme précedemment et R'₃ représente un groupe alkyle en C₁-C₁₂ linéaire ou ramifié ou un groupe alkyle linéaire ou ramifié en C₃-C₁₀ comportant une ou plusieurs liaisons C=C ou C≡ C.

La saponification des dérivés précédents de formule IX, selon les méthodes connues de l'homme de l'art, notamment par réaction avec une base forte, comme par exemple l'hydroxyde de sodium ou de potassium, dans un alcool, comme par exemple l'éthanol, conduit à l'acide a,a-diméthyl-4-pipéridineacétique correspondant que l'on amidifie par une amine appropriée selon les méthodes connues de l'homme de l'art notamment par réaction en présence de chlorure de thionyle dans un solvant halogéné, comme par exemple le trichlorométhane, pour obtenir le dérivé de α,α-diméthyl-4-pipéridineacétamide de formule IV recherché.

Les composés intermédiaires de formule IV, dans laquelle R'₁ et R'₂ identiques ou différents représentent chacun un atome d'hydrogène, un groupe alkyle linéaire ou ramifié en C₁-C₄, un groupe phénylméthyle ou forment avec l'atome d'azote auquel ils sont liés un groupe pipéridinyle et R'₃ représente un groupe alkyle en C₁-C₁₂ linéaire ou ramifié, ou un groupe alkyle linéaire ou ramifié en C₃-C₁₀ comportant une ou plusieurs liaisons C=C ou C≡C, sont des composés nouveaux et constituent un des objets de l'invention.

Les composés intermédiaires de formule IX, dans laquelle W représente un atome d'hydrogène ou un groupe alkyle en C₁-C₄ et R'₃ représente un groupe alkyle en C₁-C₁₂ linéaire ou ramifié ou un groupe alkyle linéaire ou ramifié en C₃-C₁₀ comportant une ou plusieurs liaisons C=C ou C≡C, sont nouveaux, à l'exception du composé dans lequel W et R'₃ représentent tous deux un groupe méthyle.

De même les dérivés intermédiaires de formule VI et VII, dans lesquelles W représente un groupe alkyle linéaire ou ramifié en C₁-C₄ et Z un groupe protecteur comme par exemple un groupe phénylméthyle, 4-méthoxyphénylméthyle ou COOY où Y représente un groupe alkyle en C₁-C₄ ou un groupe phénylméthyle, sont des composés nouveaux.

Les composés intermédiaires de formule VIII, dans laquelle W représente un groupe alkyle linéaire ou ramifié en C₁-C₄, sont des composés nouveaux.

L'invention sera mieux comprise à la lecture des exemples de préparation qui suivent. Ces exemples sont destinés à illustrer l'invention sans en limiter la portée. Par commodité, dans ce qui suit les "Préparations" se réfèrent à l'obtention des précurseurs et intermédiaires, et les "Exemples" se référent à l'obtention des produits de formule I selon l'invention.

### PREPARATION I

### Obtention de l'ester éthylique de l'acide 1-phénylméthyl-α,α-diméthyl-4-hydroxy-4-pipéridineacétique.

A une solution refroidie à une température de -70°C de 18,5 g (0,18 mole) de N-(1-méthyléthyl)-2-propanamine dans 75 ml de tétrahydrofuranne on ajoute goutte à goutte 125 ml de butyllithium 1,6 M. On agite pendant dix minutes à cette température puis on additionne 17,5 g (0,15 mole) de l'ester éthylique de l'acide 2-méthylpropanoïque en solution dans 70 ml de tétrahydrofuranne en maintenant la température à -70°C. Après agitation pendant une heure à cette température on ajoute une solution de 26 g (0,14 mole) de 1-phénylméthyl-4-pipéridinone dans 70 ml de tétrahydrofuranne. On agite pendant une heure et demie à une température de -70°C puis on laisse revenir la température du milieu réactionnel à la température ambiante. Après évaporation des solvants sous pression réduite, le résidu est versé dans une solution saturée de chlorure d'ammonium. La phase organique est extraite au moyen d'éther, lavée à l'eau, séchée au moyen de sulfate de magnésium, filtrée et les solvants sont évaporés sous pression réduite. Après purification par distillation on obtient 32 g (rendement : 76 %) du produit attendu, sous forme d'huile.
**Eb = 145-150°C (0,05 mm de Hg, i.e. environ 6,66 Pa)**

### PREPARATION II

### Obtention du chlorhydrate de l'ester éthylique de l'acide 1-phénylméthyl-α,α-diméthyl-1,2,3,6-tetrahydro-4-pyridineacétique.

A une solution de 50 g (16,4.10⁻² mole) de l'ester éthylique de l'acide 1-phénylméthyl-α,α-diméthyl-4-hydroxy-4-pipéridineacétique dans 200 ml de trichlorométhane et en présence de 0,5 ml de N,N-diméthylformamide on ajoute goutte à goutte 24 ml (33.10⁻² mole) de chlorure de thionyle. Le mélange réactionnel est porté à reflux pendant 18 heures puis refroidi. Les solvants sont évaporés sous pression réduite et le résidu est repris par une solution aqueuse de 20 ml d'hydroxyde de sodium 10N. On extrait la phase aqueuse avec de l'éther. La phase organique est lavée à la saumure, séchée sur sulfate de magnésium, filtrée et les solvants sont évaporés sous pression réduite. On obtient une huile que l'on reprend avec 300 ml de 2-propanol et on fait barboter dans la solution obtenue HCI gazeux jusqu'à pH acide. On filtre le précipité obtenu et le recristallise dans le 2-propanol. On obtient 38,9 g (rendement : 74 %) du produit attendu.
**F = 208°C**

### PREPARATION III

### Otention de l'ester éthylique de l'acide α,α-diméthyl-4-pipéridine-acétique.

On hydrogène dans un appareil de Parr à 50°C sous une pression d'hydrogène de 3.10⁶ Pa en présence de 20 g de charbon palladié à 5 % une solution de 246 g (76,6.10⁻² mole) de chlorhydrate de l'ester éthylique de l'acide 1-phénylméthyl-α,α-diméthyl-1,2,3,6-tetra-hydro-4-pyridineacétique dans 1,5 litre d'éthanol. Après filtration, le filtrat obtenu est évaporé sous pression réduite. On reprend le résidu obtenu par de l'eau et alcalinise avec 500 ml d'hydroxyde de sodium 5N. On extrait à l'ester éthylique de l'acide acétique. La phase organique est séchée avec du sulfate de magnésium, filtrée et les solvants sont évaporés sous pression réduite. On obtient 125,3 g (rendement : 82 %) du produit recherché sous forme d'huile.
**n**^{**21**} **= 1,47**

### PREPARATION IV

### Obtention de l'ester éthylique de l'acide 1-dodécyl-α,α-diméthyl-4-pipéridineacétique,(E)-2-buténedioate.

Un mélange de 27 g (0,135 mole) d'ester éthylique de l'acide α,α-diméthyl-pipéridineacétique, de 42,2 g (0,169 mole) de 1-bromododécane et de 46,8 g (0,339 mole) de bicarbonate de potassium dans 250 ml d'acétonitrile est chauffé au reflux pendant 5,5 h. Après avoir versé le mélange dans de la glace, la phase organique est extraite au moyen d'ester éthylique de l'acide acétique, lavée à l'eau, séchée, filtrée et les solvants sont évaporés sous pression réduite. Après purification par chromatographie sur gel de silice en éluant au moyen d'hexane/2-propanone 9/1 (v/v) on obtient 33,9 g (Rendement : 68 %) de l'ester éthylique de l'acide 1-dodécyl-α,α-diméthyl-4-pipéridineacétique sous forme d'huile. A partir de cette huile on prépare le fumarate attendu.
**F = 106°C**

### PREPARATION V

### Obtention du chlorhydrate de l'acide 1-dodécyl-α,α-diméthyl-4-pipéridineacétique.

On ajoute 24,6 g (61,4.10⁻² mole) d'hydroxyde de sodium en pastilles à une solution de 22,5 g (6.10⁻² mole) de l'ester éthylique de l'acide 1-dodécyl-α,α-diméthyl-4-pipéridineacétique dans 210 ml d'éthanol/eau 2/1 (v/v) et chauffe le mélange réactionnel à reflux pendant 72 heures. Celui-ci est ensuite versé dans 200 ml d'acide chlorhydrique 5N. Le précipité obtenu est filtré et lavé avec de l'éther. Après recristallisation dans un mélange éthanol-eau 2/8 (v/v) on obtient 17,6 g (rendement : 78 %) du produit attendu.
**F = 192°C**

### PREPARATION VI

### Obtention de 1-dodécyl-α,α-diméthyl-4-pipéridineacétamide

Une solution de 8,4 g (2,2.10⁻² mole) du chlorhydrate de l'acide 1-dodécyl-α,α-diméthyl-4-pipéridineacétique dans 50 ml de chlorure de thionyle est portée à reflux pendant 8 heures. Après évaporation des solvants sous pression réduite le résidu est solubilisé dans un mélange méthylbenzène/trichlorométhane 2/1 (v/v) puis refroidi. On ajoute alors avec précaution 80 ml d'ammoniac liquide et on laisse la solution à température ambiante pendant 72 heures. On verse le mélange dans de l'eau et on extrait avec l'ester éthylique de l'acide acétique. La phase organique est lavée à l'eau, séchée avec du sulfate de magnésium et les solvants sont évaporés sous pression réduite. Le produit obtenu est purifié par chromatographie sur gel de silice en éluant au moyen du mélange ester éthylique de l'acide acétique/méthanol/ammoniaque 9/1/0,05 (v/v/v). On obtient 5,2 g (Rendement : 69 %) de produit recristallisé dans le diisopropyle éther.
**F = 123°C**

De façon analogue à la synthèse précédente on prépare les composés suivants :
* **1-dodécyl-N,N,α,α-tétraméthyl-4-pipéridineacétamide, (E)-2-butènedioate** en remplaçant l'ammoniac par la N-méthylméthanamine ;
   **F = 190°C**
* **1-dodécyl-N-phénylméthyl-N,α,α-triméthyl-4-pipéridineacétamide, éthanedioate** en remplaçant l'ammoniac par la N-phénylméthylméthanamine ;
   **F = 121-128°C**
* **1-dodécyl-α,α-diméthyl-N,N-diéthyl-4-pipéridineacétamide** en remplaçant l'ammoniac par la N,N-diéthylamine ;
   **F = 50°C**
* **1-dodécyl-N-phénylméthyl,α,α-diméthyl-4-pipéridineacétamide** en remplaçant l'ammoniac par la phénylméthylamine ;
   **F = 74°C**
* **1-dodécyl-N,α,α-triméthyl-4-pipéridineacétamide** en remplaçant l'ammoniac par la méthylamine ;
   **F = 78°C**
* **1-dodécyl-N-propyl-α,α-diméthyl-4-pipéridineacétamide** en remplaçant l'ammoniac par la propanamine ;
   **F = 72-75°C**
* **1-dodécyl-N-(1-méthyléthyl)-α,α-diméthyl-4-pipéridineacétamide** en remplaçant l'ammoniac par la 1-méthyléthanamine ;
   **F = 68°C**
* **1-dodécyl-N-éthyl-α,α-diméthyl-4-pipéridineacétamide** en remplaçant l'ammoniac par l'éthanamine ;
   **F = 67°C**

### Exemple 1

### Obtention de 1-dodécyl-β,β-diméthyl-4-pipéridineéthanamine, (E)-2-butènedioate

A une solution de 2,95 g (8,7.10⁻² mole) de 1-dodécyl-α,α-diméthyl-4-pipéridineacétamide dans 80 ml de méthylbenzène on ajoute goutte à goutte 10 ml d'hydrure de bis(2-méthoxyéthoxy)aluminium-sodium. Après agitation durant 1 heure à température ambiante on porte à reflux pendant 3 heures. Après refroidissement du mélange on ajoute avec précaution 50 ml d'hydroxyde de sodium 3N et on agite pendant 24 heures. On extrait au méthylbenzène. La phase organique est lavée à l'eau, séchée avec du sulfate de magnésium, filtrée et les solvants sont évaporés sous pression réduite. Le produit est purifié par chromatographie sur gel de silice en éluant au moyen du mélange ester éthylique de l'acide acétique/méthanol/ammoniaque 4/1/0,1 (v/v/v). On obtient 1,8 g (rendement : 64 %) de 1-dodécyl-β,β-diméthyl-4-pipéridineéthanamine sous forme d'huile. A partir de cette huile on obtient le (E)-2-butènedioate dans un mélange acétone-éther.
**F = 100-120°C**

En opérant selon le procédé de l'exemple 1, à partir des dérivés analogues au 1-dodécyl-α,α-diméthyl-4-pipéridineacétamide, tels que préparés précédemment, on obtient les produits suivants :

### Exemple 2

* **1-dodécyl-N,N,β,β-tétraméthyl-4-pipéridineéthanamine, (E)-2-butènedioate.**
   **F = 130°C**

### Exemple 3

* **1-dodécyl-N-phénylméthyl-N,β,β-triméthyl-4-pipéridineéthanamine, (E)-2-butènedioate.**
   **F = 124-125°C**

### Exemple 4

* **1-dodécyl-N,N-diéthyl,β,β-diméthyl-4-pipéridineéthanamine, (E)-2-butènedioate.**
   **F = 110-115°C**

### Exemple 5

* **1 -dodécyl-N-phénylméthyl,β,β-diméthyl-4-pipéridineéthanamine, (E)-2-butènedioate.**
   **F = 175-177°C**

### Exemple 6

* **1-dodécyl-N,β,β-triméthyl-4-pipéridineéthanamine, 4-méthylbenzènesulfonate.**
   **F =139°C**

### Exemple 7

* **1-dodécyl-N-propyl,β,β-diméthyl-4-pipéridineéthanamine, éthanedioate.**
   **F = 186-188°C**

### Exemple 8

* **1-dodécyl-N-(1-méthyléthyl)-,β,β-diméthyl-4-pipéridineéthanamine, 4-méthylbenzènesulfonate.**
   **F = 115-118°C**

### Exemple 9

* **1-dodécyl-N-éthyl,β,β-diméthyl-4-pipéridineéthanamine, (E)-2-butènedioate.**
   **F = 135-157°C**

### Exemple 10

### Obtention de N-méthyl-N-[2-méthyl-2-[(1-dodécyl)-4-pipéridinyl]propyl]acétamide, 4-méthylbenzènesulfonate

A une solution de 8 g (2,4.10⁻² mole) de 1-dodécyl-N,β,β-triméthyl-4-pipéridineéthanamine dans 100 ml de dichlorométhane et 16,8 ml de N,N-diéthyléthanamine on ajoute 3,4 ml (3,6.10⁻² mole) d'anhydride acétique. On agite le mélange réactionnel à température ambiante pendant 12 heures. On verse la solution dans une solution d'hydroxyde de sodium 1N. On extrait au trichlorométhane. La phase organique est lavée à l'eau, séchée, filtrée et les solvants sont évaporés sous pression réduite. On obtient 8,7 g de N-méthyl-N-[2-méthyl-2-[(1-dodécyl)-4-pipéridinyl]propyl]acétamide (rendement : 97 %) à partir duquel on prépare le 4-méthylbenzènesulfonate que l'on recristallise dans l'ester éthylique de l'acide acétique.
**F = 102-103°C**

En procédant de manière analogue en partant de 1-dodécyl-β,β-diméthyl-4-pipéridine-éthanamine on obtient le composé suivant :

### Exemple 11

**N-[2-méthyl-2-[(1-dodécyl)-4-pipéridinyl]propyl]acétamide, (E)-2-butènedioate**
**F = 125°C**

### PREPARATION VII

### Obtention de N,N,β,β-tétraméthyl-4-pyridineéthanamine

A une solution de 100 g (82,5.10⁻² mole) de (1-méthyléthyl)-4-pyridine dans 800 ml d'acide acétique et 200 ml d'une solution aqueuse à 37 % de formaldéhyde on ajoute goutte à goutte 300 ml (2,5 moles) de N,N-diméthylamine et on porte le mélange à reflux pendant 72 heures. On ajoute alors 100 ml d'une solution aqueuse à 37 % de formaldéhyde et 150 ml de N,N-diméthylamine et agite à nouveau à la température de reflux durant 24 heures. Les solvants sont évaporés sous pression réduite et le résidu est repris par une solution aqueuse à 30 % d'hydroxyde de sodium. On extrait à l'ester éthylique de l'acide acétique, la phase organique est lavée à l'eau, séchée avec du sulfate de magnésium, filtrée et les solvants sont évaporés sous pression réduite. On récupère 70,5 g du produit attendu (rendement : 49 %) sous forme d'huile.
**Eb = 50°C sous 0,05 mm de mercure (i.e. environ 6,66 Pa)**

De façon analogue à la synthèse précédente on prépare le composé 4-[[1-(1-pipéridinyl)-2-méthyl]propyl]pyridine, (E)-2-butènedioate en remplaçant la N,N-diméthylamine par la pipéridine.
**F = 160°C**

### PREPARATION VIII

### Obtention de N,N,β,β-tétraméthyl-4-pipéridineéthanamine

On hydrogène dans un appareil de Parr à 50°C sous une pression d'hydrogène de 3.10⁶ Pa en présence de dioxyde de platine une solution de 2 g (1,1.10⁻² mole) de N,N,β,β-tétraméthyl-4-pyridine-éthanamine dans 400 ml d'acide acétique. Après élimination du catalyseur par filtration on évapore les solvants sous pression réduite. On reprend le résidu obtenu par une solution aqueuse d'hydroxyde de sodium à 30 % et on extrait au chlorure de méthylène. La phase organique est lavée, séchée avec du sulfate de magnésium, filtrée et les solvants sont évaporés sous pression réduite. On obtient 1,9 g (rendement : 92 %) du composé attendu sous forme d'huile.
**Eb = 60-64°C sous 0,02 mm de mercure (i.e. environ 2,66 Pa)**

De la même façon en partant du composé 4-[[1-(1-pipéridinyl)-2-méthyl]propyl]pyridine on prépare le composé 1-[2-(4-pipéridinyl)-2-méthyl]propyl]pipéridine
**Eb = 98-100°C sous 0,02 mm de mercure (i.e. environ 2,66 Pa)**

### Exemple 2 (autre voie de synthèse)

### Obtention de 1-dodécyl-N,N,β,β-tétraméthyl-4-pipéridineéthanamine, (E)-2-butènedioate

On porte à reflux durant 12 heures un mélange de 13 g (7.10⁻² mole) de N,N,β,β-tétraméthyl-4-pipéridineéthanamine, 19,7 g (7,9.10⁻² mole) de 1-bromododécane, 24,4 g (17,6.10⁻² mole) de carbonate de potassium et 0,5 g d'iodure de potassium dans 150 ml d'acétonitrile.

Après avoir versé le mélange réactionnel dans de l'eau on extrait à l'ester éthylique de l'acide acétique. La phase organique est lavée à l'eau, séchée avec du sulfate de magnésium, filtrée, et les solvants sont évaporés sous pression réduite. On prépare le (E)-2-butènedioate dans l'éthanol. On obtient 23,6 g (rendement : 51 %) du produit attendu après recristallisation dans le mélange 2-propanol/eau 95/5 (v/v).
**F = 130°C**

De façon analogue à la synthèse précédente on prépare les produits suivants :

### Exemple 12

* **N,N,β,β,1-pentamèthyl-4-pipéridineéthanamine, (E)-2-butènedioate.**
   **F = 194-195°C**

### Exemple 13

* **1-(2-propènyl)-N.N,β,β-tétraméthyl-4-pipéridineéthanamine, (Z)-2-butènedioate.**
   **F = 147°C**

### Exemple 14

* **1-(3,3-diméthylpropèn-2-yl)-N,N,β,β-tétraméthyl-4-pipéridine-éthanamine, (E)-2-butènedioate.**
   **F = 184°C**

### Exemple 15

* **1-[[2-[4-(1-dodécylpipèridinyl)]-2-méthyl]propyl]pipéridine, (Z)-2-butènedioate.**
   **F = 129°C**

### Exemple 16

### Obtention de (E)-1-(6,6-diméthyl-2-heptèn-4-yn-1-yl)-N,N β,β-tétraméthyl-4-pipéridineéthanamine, (Z)-2-butènedioate

On ajoute goutte à goutte 12 g (6.¹⁰⁻² mole) de 1-bromo-6,6-diméthyl-2-hepten-4-yne (E/Z = 3/1) en solution dans 20 ml de N,N-diméthylformamide à un mélange de 10 g (5,4.10⁻² mole) de N,N,β,β-tétraméthyl-4-pipéridineéthanamine et 14,9 g de carbonate de potassium dans 150 ml de N,N-diméthylformamide. On agite le mélange pendant 48 heures à température ambiante puis on le verse dans de l'eau. On extrait en plusieurs fois à l'ester éthylique de l'acide acétique. La phase organique est lavée à l'eau, séchée avec du sulfate de magnésium, filtrée et les solvants sont évaporés sous pression réduite. On purifie l'huile résiduelle obtenue par chromatographie sur une colonne de silice en éluant au moyen du mélange chlorure de méthylène/méthanol 9/1 (v/v). Après évaporation des solvants sous pression réduite on obtient 10,3 g (rendement : 56 %) de (E)-1-(6,6-diméthyl-2-heptèn-4-yn-1-yl)-N,N,β,β-tétraméthyl-4-pipéridineéthanamine. A partir de ce dernier on prépare le (Z)-2-butènedioate correspondant dans l'éthanol.
**F = 185-188°C**

### Exemple 17

### Obtention de 1-(6,6-diméthyl-heptyl)-N,N,β,β-tétraméthyl-4-pipéridine-éthanamine, (E)-2-butènedioate

On hydrogène dans un appareil de Parr à 40°C, sous une pression d'hydrogène de 3.10⁵ Pa, en présence de 0,5 g de charbon palladié, 4,5g (1,5.10⁻² mole) de 1-(6,6-diméthyl-2-heptèn-4-yn-1-yl)-N,N,β,β-tétraméthyl-4-pipéridineéthanamine en solution dans 150 ml de méthanol, sous forme de mélange d'isomères E/Z 3/1. Lorsque la réaction est terminée, on élimine le catalyseur par filtration et les solvants sont évaporés sous pression réduite. On obtient 3,8 g de produit (rendement : 84 %) sous forme d'huile. A partir de cette huile on prépare le (E)-2-butènedioate dans l'éthanol
**F = 161-166°C**

### Exemple 18

### Obtention de 1[3-[4-(méthyléthyl)phényl]-2-méthyl-propyl]-N,N,β,β-tétraméthyl-4-pipéridineéthanamine, (E)-2-butènedioate

A une solution de 6,7 g (3,6.10⁻² mole) de N,N,β,β-tétraméthyl-4-pipéridineéthanamine dans 100 ml de méthanol on ajoute 7,6 g (4.10⁻² mole) de 2-méthyl-3-[4-(méthyléthyl)phényl]propanol et 5,1 g de sulfate de sodium. Après addition d'acide acétique pour ajuster le pH du mélange à 6,5 on ajoute 2,6 g (4.10⁻² mole) de cyanoborohydrure de sodium et agite ce mélange réactionnel pendant 32 heures. Après filtration et évaporation des solvants, on reprend le résidu par une solution normale d'hydroxyde de sodium. On extrait à l'éther. La phase organique est lavée à l'eau, séchée, filtrée et les solvants sont évaporés sous pression réduite.

Le produit obtenu est purifié par chromatographie sur une colonne de silice en éluant au moyen du mélange chlorure de méthylène/méthanol. On récupère 5 g d'un produit sous forme d'huile (rendement : 40 %). A partir de ce cette huile on prépare le (E)-2-butènedioate dans l'éthanol.
F = 146-150°C

### PREPARATION IX

### Obtention de 1-(11-bromoundécanoyl)-N,N,β,β-tétraméthyl-4-pipéridineéthanamine

A une solution refroidie à 0°C de N,N,β,β-tétraméthyl-4-pipéridineéthanamine dans 120 ml de trichlorométhane et 19,2 ml (14,7.10⁻² mole) de N,N-diéthyléthanamine, on ajoute goutte à goutte 17,5 g (6,2.10⁻² mole) de chlorure de l'acide 11-bromo-undecanoïque dilué dans 100 ml de trichlorométhane. On agite le mélange réactionnel pendant 3 heures à 0°C puis 20 heures à température ambiante. On verse le mélange dans de l'eau et laisse décanter. La phase organique est récupérée, séchée avec du sulfate de magnésium et les solvants sont évaporés sous pression réduite. On obtient 12 g (rendement : 57 %) du produit attendu sous forme d'huile.

De la même façon à partir du chlorure de l'acide undecanoïque on prépare le produit suivant :

### Exemple 19

**1-undécanoyl-N,N,β,β-tétraméthyl-4-pipéridineéthanamine, éthanedioate.**
**F = 134°C**

### Exemple 20

### Obtention de 1-[11-(imidazolyl)undécanoyl]-N,N β,β-tétraméthyl-4-pipéridineéthanamine, éthanedioate

On ajoute 1,8 g 12,6.10⁻² mole) d'imidazole à une suspension de 0,62 g d'hydrure de sodium à 60 % dans 200 ml de N,N-diméthylformamide et on agite une heure à température ambiante. On ajoute alors 11 g (2,5.10⁻² mole) du 1-(11-bromodécanoyl)-N,N,β,β-tétraméthyl-4-pipéridineéthanamine obtenu sous forme d'huile précédemment, dilué dans 100 ml de N,N-diméthylformamide et on chauffe le mélange à 90°C pendant 4 heures. Après évaporation des solvants, le résidu est repris avec de l'eau. On extrait à l'éther, la phase organique est lavée à l'eau, séchée, filtrée et les solvants sont évaporés sous pression réduite. Après purification par chromatographie sur colonne de silice en éluant au moyen du mélange chlorure de méthylène/méthanol 9/1 (v/v), on obtient 6 g (rendement : 57 %) de produit sous forme d'huile. A partir de cette huile on prépare l'éthanedioate dans l'acétone.
F = 75-80°C

### Exemple 21

### Obtention de 1-[(11-imidazolyl)undecyl]-N,N,β,β-tétraméthyl-4-pipéridineéthanamine, (E)-2-butènedioate

A une solution de 4 g (0,9.10⁻² mole) de 1-undécanoyl-N,N,β, β-tétraméthyl-4-pipéridineéthanamine, dans 120 ml de méthylbenzène, on ajoute goutte à goutte 10,5 ml (3,8.10⁻² mole) d'hydrure de bis-(2-méthoxyéthoxy)aluminium sodium et on chauffe le mélange à reflux pendant 4 heures. On ajoute alors goutte à goutte 100 ml d'une solution d'hydroxyde de sodium 3N et agite le mélange 24 heures à température ambiante. On extrait à l'aide de méthylbenzène. La phase organique est lavée avec une solution d'hydroxyde de sodium normale puis avec de l'eau, séchée, filtrée et les solvants sont évaporés sous pression réduite. On obtient 2,8 g (rendement : 74 %) de 1-[(11-imidazolyl)undecyl]-N,N, β,β-tétraméthyl-4-pipéridineéthanamine sous forme d'huile. A partir de cette huile on prépare le (E)-2-butènedioate dans l'éthanol.
**F = 105-110°C**

### Exemple 22

### Obtention de 2-[[4-[2-(1-diméthylamino-2-méthyl)propyl]-1-pipéridinyl]propyl]-1H-isoindole-1,3(2H)-dione, (E)-2-butènedioate

On porte à reflux pendant 5 heures un mélange de 15 g (8.10⁻² mole) de N,N,β,β-tétraméthyl-4-pipéridineéthanamine, 24 g (9.10⁻² mole) de 2-(3-bromopropyl)-1H-isoindole-1,3(2H)-dione, 28 g de carbonate de potassium et quelques cristaux d'iodure de sodium dans 150 ml d'acétonitrile. Après avoir versé le mélange réactionnel dans l'eau on extrait avec l'ester éthylique de l'acide acétique. La phase organique est lavée à l'eau, séchée avec du sulfate de magnésium, filtrée et les solvants sont évaporés sous pression réduite. Le résidu d'évaporation est purifié par chromatographie sur colonne de silice en éluant au moyen du mélange ester éthylique de l'acide acétique/méthanol 99/1 (v/v). On récupère 5,9 g de 2-[[4-[2-(1-diméthylamino-2-méthyl)propyl]-1-pipéridinyl]propyl]-1H-isoindole-1,3(2H)-dione sous forme d'huile. A partir de cette huile on prépare le (E)-2-butènedioate dans l'éthanol.
F = 124°C

### Exemple 23

### Obtention de 1-[1-(3-aminopropyl-N,N,β,β-tétraméthyl-4-pipéridine éthanamine, (E)-2-butènedioate

On porte à reflux une solution de 14 g (3,8.10⁻² mole) de 2-[[4-[2-1-diméthylamino-2-méthyl)propyl]-1-pipéridinyl]propyl]-H-isoindole-1,3(2H)-dione] dans 100 ml d'éthanol à 95 % et 4 ml d'hydrate d'hydrazine. Le mélange est versé dans une solution d'hydroxyde de sodium 5N et on extrait avec l'ester éthylique de l'acide acétique. La phase organique est lavée à l'eau, séchée avec du sulfate de magnésium, filtrée et les solvants sont évaporés sous pression réduite. On obtient 7,9 g (rendement : 86 %) de 1-[1-(3-aminopropyl)]-N,N,β,β-tétraméthyl-4-pipéridineéthanamine sous forme d'huile jaune. A partir de cette huile on prépare le (E)-2-butènedioate dans l'éthanol.
F = 189-191°C

### Exemple 24

### Obtention de N-[[4-[2-(1-diméthylamino-2-méthyl)propyl]-1-pipéridinyl]-1-propyl]-5-méthylhexanamide, éthanedioate

On solubilise 4,2 g (1,7.10⁻² mole) de 1-[1-(3-aminopropyl)]-N,N,β,β-tétraméthyl-4-pipéridineéthanamine dans 50 ml de chlorure de méthylène et 12 ml de N,N-diéthyléthanamine. On refroidit la solution à 0°C et additionne goutte à goutte 2,6 g (1,7.10⁻² mole) de chlorure de l'acide 5-méthylhexanoïque en solution dans 25 ml de chlorure de méthylène. On laisse la température du mélange revenir à 20°C et continue l'agitation durant 12 heures à cette température. On verse le mélange dans de l'eau et on extrait au chlorure de méthylène. La phase organique est lavée à l'eau, séchée avec du sulfate de magnésium, filtrée et les solvants sont évaporés sous pression réduite. Le résidu d'évaporation est purifié par chromatographie sur colonne de silice en éluant au moyen du mélange chlorure de méthylène/méthanol 95/5 (v/v). On récupère 2 g (rendement : 32 %) de N-[[4-[2-(1-diméthylamino-2-méthyl)propyl]-1-pipéridinyl]-1-propyl]-5-méthylhexanamide sous forme d'huile. A partir de cette huile on prépare dans l'acétone l'éthanedioate recherché.
**F = 73-75°C**

### Exemple 25

### Obtention de l'ester éthylique de l'acide 4-[2-[(1-diméthylamino-2-méthyl)propyl]-1-pipéridinebutanoïque (Z)-2-butènedioate

On porte à reflux pendant 4 heures un mélange de 12 g (6,5.10⁻² mole) de N,N,β,β-tétraméthyl-4-pipéridineéthanamine, 11,6 ml (8.10⁻² mole) d'ester éthylique de l'acide 4-bromobutanoïque et de 22,4 g de carbonate de potassium dans 200 ml d'acétonitrile. Après évaporation des solvants sous pression réduite on reprend le résidu avec de l'eau. On extrait à l'ester éthylique de l'acide acétique. La phase organique est lavée à l'eau, séchée avec du sulfate de magnésium, filtrée et les solvants sont évaporés sous pression réduite. Après purification par chromatographie sur colonne de silice en éluant au moyen du mélange chlorure de méthylène/méthanol 9/1 (v/v) on récupère 11,8 g (rendement : 61 %) de l'ester éthylique de l'acide 4-[2-[(1-diméthylamino-2-méthyl)propyl]-1-pipéridinebutanoïque sous forme d'huile. A partir de cette huile on prépare dans l'éthanol le (Z)-2-butènedioate correspondant.
**F = 122°C**

### Exemple 26

### Obtention de N-(4-méthylpentyl)-4-[2-(1-diméthylamino-2-méthyl)propyl]-1-pipéridinebutanamide,(E)-2-butènedioate

Une solution de 4 g (1,2.10⁻² mole) de l'ester éthylique de l'acide 4-[2-[(1-diméthylamino-2-méthyl)propyl]-1-pipéridinebutanoïque et de 9 g (9.10⁻² mole) de 4-méthylpentanamine dans 50 ml de méthylbenzène est introduite dans un autoclave et chauffée à 200°C pendant 96 heures. Après évaporation du méthylbenzène sous pression réduite le résidu est purifié par chromatographie sur colonne de silice en éluant au moyen du mélange chlorure de méthylène/méthanol 95/5 (v/v). On récupère après évaporation sous pression réduite des fractions pures 3 g (rendement : 71 %) de N-(4-méthylpentyl)-4-[2-(1-diméthylamino-2-méthyl)propyl]-1-pipéridinebutanamide sous forme d'huile. A partir de cette huile on prépare dans l'éthanol le (E)-2-butènedioate correspondant.
**F = 140°C**

### Exemple 27

### Obtention du iodure de 1-[(1-undecyl)carbonyl]-N,N,N,β,β-pentaméthyl-4-pipéridineéthanaminium

On porte à reflux en agitant une solution de 1 g (0,27.10⁻² mole) de 1-undecanoyl-N,N,β,β-tétraméthyl-4-pipéridineéthanamine, de 20 ml d'éther et de 2 ml d'iodométhane. Au bout de 15 heures on ajoute à nouveau 2 ml d'iodométhane et encore 3 ml 24 heures après et on chauffe encore durant environ 8 heures. Puis on refroidit le mélange et filtre le précipité qui s'est formé. On obtient ainsi 0,9 g (rendement : 65 %) d'un produit blanc correspondant au composé recherché.
**F = 208°C**

En procédant de manière analogue à la préparation IX, on obtient les produits suivants :

### Exemple 28 :

**1-(3-phénylpropènoyl)-N,N,β,β-tétraméthyl-4-pinéridineéthanamine, (E)-2-buténedioate.**
**F = 173-176° C**

### Exemple 29

**1-[2(4-chlorophénoxy)-2,2.diméthyl-éthanoyl]-N,N,β,β-tétraméthyl-4-**-**pipéridineéthanamine, (E)-2-butènedioate.**
**F = 194° C**

En procédant de manière analogue à la préparation de l'exemple 1, on obtient les produits suivants :

### Exemple 30 :

### 1-[2-(4-chlorophénoxy)-2,2-diméthyl-éthyl]-N,N,β,β-tétraméthyl-4- pipéridineéthanamine, (E)-2-butènedioate.

**F = 103° C**

### Exemple 31 :

### 1-(3-phénylpropènyl)-N,N,β,β-tétraméthyl-4-pipéridineéthanamine, (E)-2-butènedioate.

**F = 150° C**

### Exemple 32 :

### 1-[2-[4-(2-méthylpropyl)phényl]-2-méthyléthyl]-N,N,β,β-tétraméthyl-4-pipéridineéthanamine, (E)-2-butènedioate.

**F = 147-150° C**

On a regroupé dans le tableau I suivant un certain nombre de composés selon l'invention. Dans ce tableau les symboles utilisés ont les significations suivantes:
- n-Pr :: -CH₂-CH₂-CH₃
- n-C₁₂ :: -(CH₂)₁₁CH₃
- i-Pr:: -CH(CH₃)₂
- Bn:: 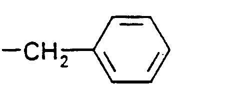
- Ph:: 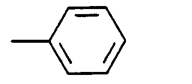
- p-ClPh:: 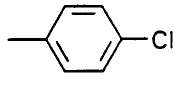
- Tbu :: -C(CH₃)₃
- Pht:: 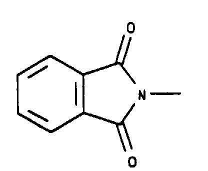
- Im:: 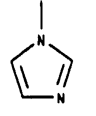
- A:: 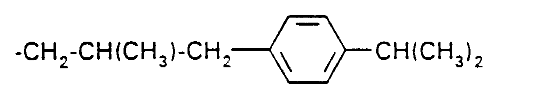
- B :: -(CH₂)₃-NH-CO-(CH₂)₃-CH(CH₃)₂
- C :: -(CH₂)₃-CO-NH-(CH₂)₃-CH(CH₃)₂
- D:: -CH₂CH=CH-C≡C(CH₃)₃ (E)
- F::

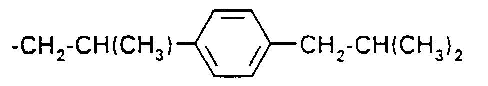

Les symboles utilisés pour les sels ont les significations suivantes :
- oxal:: HO₂C-CO₂H
- Fum :: HO₂C-CH=CH-CO₂H (E)
- Mal :: HO₂C-CH=CH-CO₂H (Z)
- TSO₃H:: 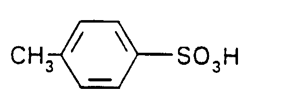
- IMA :: iodure de méthylammonium

Les produits selon l'invention sont des inhibiteurs de la biosynthèse du cholestérol et notamment de l'époxysqualène cyclase.

L'activité des composés selon l'invention a été évaluée par mise en évidence d'un effet inhibiteur sur l'époxysqualène cyclase des microsomes hépatiques de rats mâles Wistar.

La méthode consiste à mesurer le lanostérol formé à partir du R,S-2,3-oxydosqualène par l'enzyme microsomiale. La préparation de l'enzyme se fait selon la méthode décrite par Ness G.C. (Ness G.C. et al., Biochem. J., (1986) 233,167-172).

### Méthode de mesure de l'activité époxysqualène cyclase:

Les microsomes hépatiques de rat sont utilisés comme source d'enzyme. La méthode consiste à mesurer le lanostérol formé à partir du R,S-2,3-oxydosqualène. Le R,S-2,3-oxydosqualène, les produits à tester et le TWEEN® 80 sous forme de solutions organiques (2-propanone ou sulfinylbis-méthane) (25 µl) sont mis dans les tubes d'essai puis 400 µl de tampon phosphate de potassium (0,1 M, pH = 7,4) sont ajoutés. La réaction est initiée par addition de 100 µl de microsomes. Pour un volume réactionnel final de 525 µl, le mélange contient 150 µM de R,S-2,3-oxydosqualène, 0,1 % de TWEEN® 80 (pour solubiliser le R,S-2,3-oxydosqualène) et 250 µg de protéines microsomiales. La durée de réaction est de 60 minutes à 37°C. La réaction est stoppée par addition de 300 µl de potasse méthanolique (7 %) et de 20 µg de stigmastérol comme standard interne. Après saponification à 80°C pendant 30 minutes et agitation à l'agitateur rotatif, les stérols sont extraits au moyen de 2 ml d'hexane. Le lanostérol formé est séparé du R,S-2,3-oxydosqualéne, du cholestérol membranaire et du stigmastérol par chromatographie en phase gazeuse après transformation en éthers triméthylsilylés. La dérivatisation des stérols s'effectue à 60°C pendant 30 minutes après addition de 25 µl de pyridine et 75 µl de l'ester triméthylsilique de l'acide 2,2,2-trifluoro-N-triméthylsilyl-éthanimidique contenant 1 % d'éthers triméthylchlorosilanes.

Après évaporation, les éthers triméthylsilyles sont remis en solution dans 100 µl d'hexane. Une partie aliquote de cette solution (2 µl) est chromatographiée en phase gazeuse sur colonne capillaire OV1 (0,32 mm, 25 m) dans les conditions suivantes : température de l'injecteur = 270°C, température du four = 260°C, température du détecteur = 300°C, le gaz vecteur étant l'azote à une pression de 7.10⁴ Pa.

La puissance des molécules testées est exprimée en pourcentage d'inhibition de la quantité de lanostérol formé pour une concentration de 25.10⁻⁶ mole par litre du produit testé. Les résultats obtenus avec un certain nombre de composés selon l'invention sont regroupés dans le tableau II.

Les produits selon l'invention sont utiles en thérapeutique dans le traitement et la prévention de l'hypercholestérolémie, notamment des phénomènes de lésions artérielles qui y sont associés tel l'athérosclérose, et des mycoses et autres affections parasitaires provoquées par un champignon comme par exemple ***Actinomyces mentagrophytes, Candida tropicalis, Candida albicans, Candida glabrata*** ou ***Aspergillus fumigatus.***

Selon l'invention on préconise une composition thérapeutique caractérisée en ce qu'elle renferme au moins un composé de formule I ou l'un de ses sels d'addition en quantité thérapeutiquement efficace, en association avec un excipient physiologiquement acceptable.

On préconise également l'utilisation des composés de formule I ou l'un de leurs sels d'addition, en tant qu'agents inhibiteurs de l'époxysqualène cyclase, pour l'obtention d'un médicament préventif ou curatif hypocholestérolémiant, hypolipémiant, antiathéromateux et/ou antifongique. Les produits de formule I selon l'invention et leurs sels d'addition sont en particulier utiles dans le traitement des D.I.C. (coagulations intravasculaires disséminées) induites notamment par les moisissures telles que ***Candida albicans*** et ***Candida glabrata.***

Le meilleur mode de mise en oeuvre de l'invention consiste à utiliser les produits des exemples 2, 16 et 18 en tant que médicaments notamment hypocholestérolémiants et/ou antifongiques.

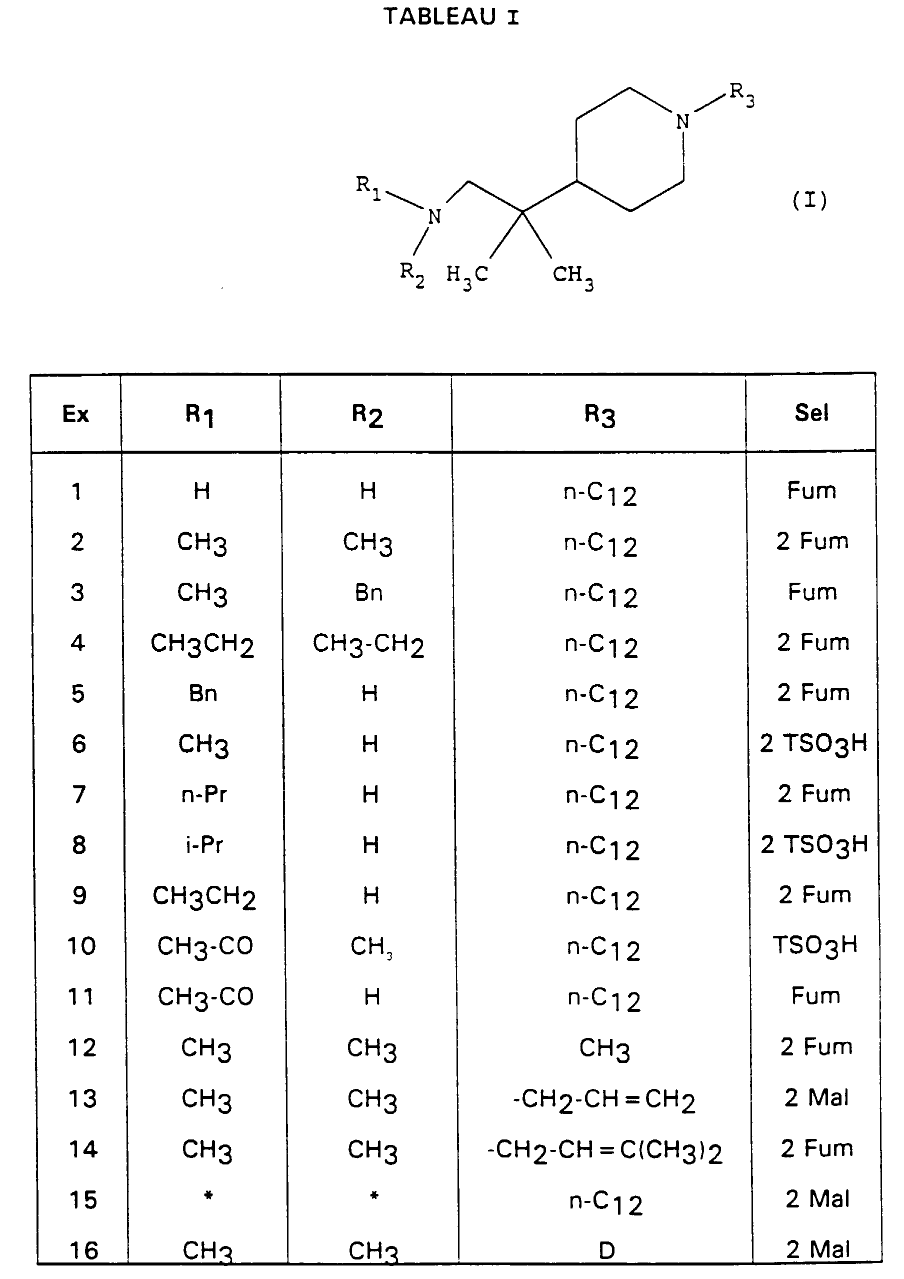

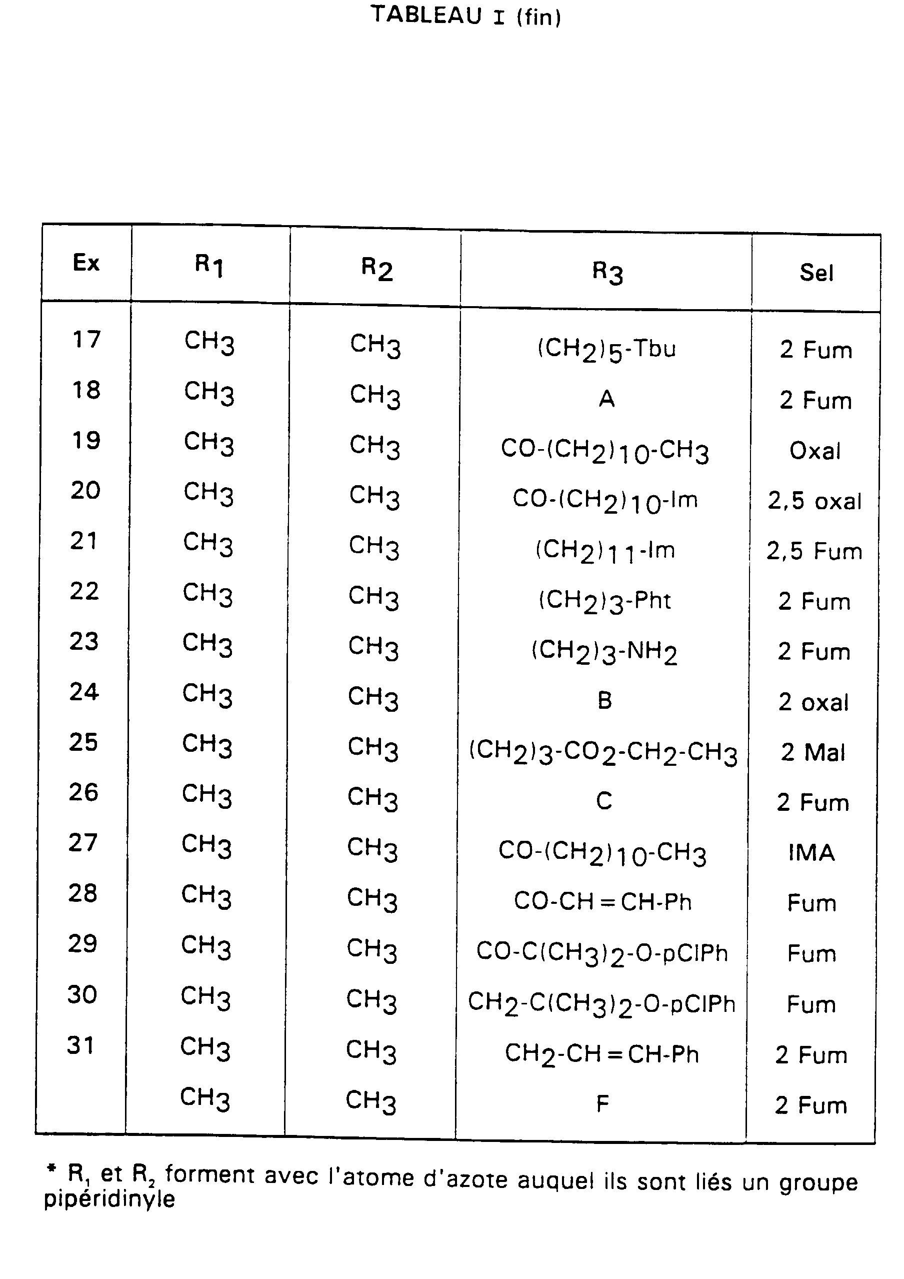

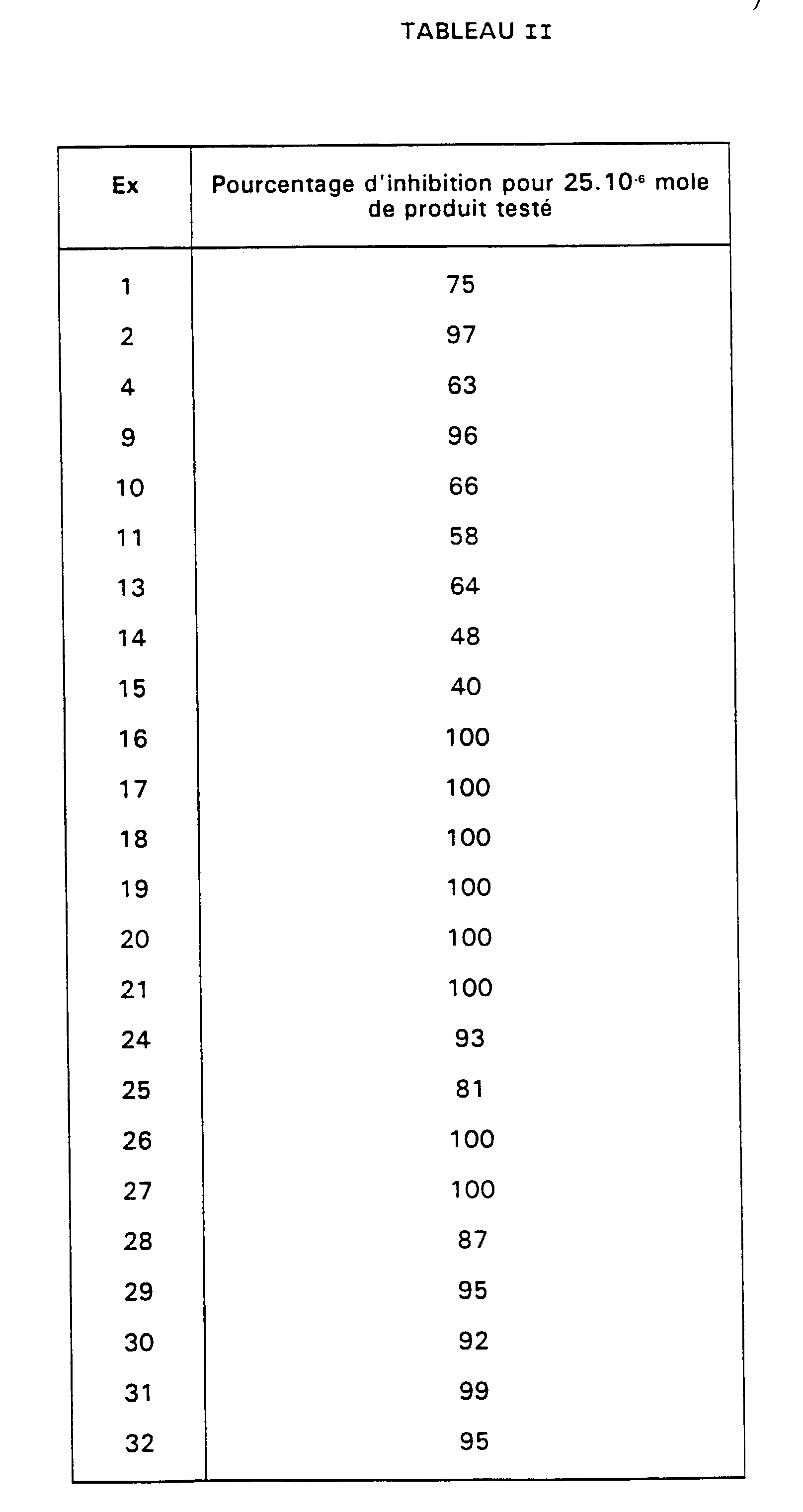

## Revendications

1. Composé β,β-diméthyl-4-pipéridineéthanamine caractérisé en ce qu'il est choisi parmi l'ensemble constitué par :
(i) les composés de formule : 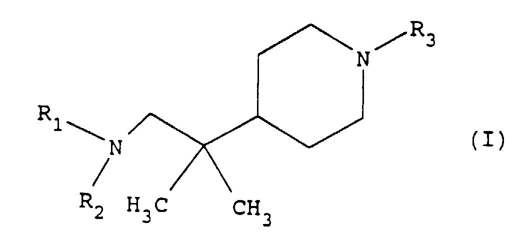 dans laquelle
- R₁ et R₂, identiques ou différents, représentent chacun l'atome d'hydrogène, un groupe alkyle en C₁-C₄, un groupe acyle en C₂-C₅, un groupe phénylméthyle ou forment avec l'atome d'azote auquel ils sont liés un groupe pipéridinyle,
- R₃ représente
- un groupe alkyle en C₁-C₁₂ linéaire ou ramifié, éventuellement substitué par :
a) un groupe imidazolyle ou
b) un groupe phényle lui-même éventuellement substitué par un groupe alkyle linéaire ou ramifié en C₁-C₄,
- un groupe alkyle linéaire ou ramifié en C₃-C₁₀ comportant une ou plusieurs liaisons C=C ou C≡C, et éventuellement substitué par un groupe phényle,
- un groupe alkyloxy en C₁-C₄, linéaire ou ramifié, substitué par un groupe para-chlorophényle,
- un groupe -CO-R₈, dans lequel R₈ représente un groupe alkyle linéaire ou ramifié en C₈-C₁₁ éventuellement substitué par un groupe imidazolyle, un groupe alkyloxy en C₁-C₄, linéaire ou ramifié, substitué par un groupe para-chlorophényle ou un groupe alkyle en C₂-C₄, linéaire ou ramifié, comportant au moins une liaison double, et substitué par un groupe phényle,
- un groupe alkyle linéaire ou ramifié en C₁-C₄ substitué par l'un des groupes suivants :
un groupe -COOR₄, dans lequel R₄ représente soit un atome d'hydrogène soit un groupe alkyle linéaire ou ramifié en C₁-C₄,
un groupe 1H-isoindole-1,3(2H)-dione,
un groupe NR₅R₆, dans lequel R₅ et R₆, identiques ou différents, représentent chacun un atome d'hydrogène ou un groupe alkyle linéaire ou ramifié en C₁-C₄,
un groupe -NH-CO-R₇ ou un groupe -CO-NH-R₇ dans lesquels R₇ représente un groupe alkyle linéaire ou ramifié en C₁-C₆ ; et,
(ii) leurs sels d'addition, notamment les sels d'addition d'acide et les sels d'ammonium.

2. Composé selon la revendication 1 caractérisé en ce que le sel d'addition est un sel obtenu à partir d'un acide organique ou minéral.

3. Composé de formule I selon la revendication 1 caractérisé en ce que le sel d'addition est un sel d'ammonium obtenu à partir d'un iodure d'alkyle en C₁-C₁₄.

4. Composé selon la revendication 1 caractérisé en ce qu'il s'agit du sel 1 dodécyl-N,N,β,β-tétraméthyl-4-pipéridineéthanamine, (E)-2-butènedioate.

5. Composé selon la revendication 1 caractérisé en ce qu'il s'agit du sel (E)-1-(6,6-diméthyl-2-hepten-4-yn-1-yl)-N,N,β,β-tétraméthyl-4-pipéridineéthanamine, (Z)-2-butènedioate.

6. Composé selon la revendication 1 caractérisé en ce qu'il s'agit du sel 1-[3-[4-(méthyléthyl)phényl]-2-méthyl-propyl]-N,N,β,β-tétraméthyl-4-pipéridineéthanamine, (E)-2-butènedioate.

7. Procédé de préparation d'un composé de formule I ou de l'un de ses sels selon la revendication 1, ledit procédé étant caractérisé en ce qu'il comprend les étapes consistant à :
(i) soumettre à une réaction de N-alkylation ou N-acylation un composé de formule : 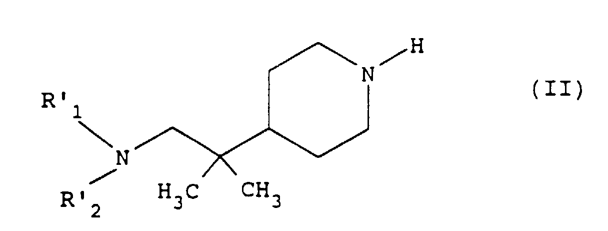 dans laquelle :
R'₁ et R'₂, identiques ou différents, représentent chacun un atome d'hydrogène, un groupe alkyle en C₁-C₄, un groupe phénylméthyle ou forment avec l'atome d'azote auquel ils sont liés un groupe pipéridinyle, par réaction avec un composé de formule R'₃-X où X représente un halogène comme par exemple un atome de brome ou de chlore et R'₃ représente :
un groupe alkyle en C₁-C₁₂ linéaire ou ramifié éventuellement substitué par un groupe imidazolyle ou un groupe phényle lui-même éventuellement substitué par un groupe alkyle linéaire ou ramifié en C₁-C₄,
un groupe alkyle linéaire ou ramifié en C₃-C₁₀ comportant une ou plusieurs liaisons double ou triple,
un groupe -CO-R'₈ dans lequel R'₈ représente un groupe alkyle linéaire ou ramifié en C₈-C₁₁ éventuellement substitué par un groupe imidazolyle,
un groupe alkyle linéaire ou ramifié en C₁-C₄ substitué par
un groupe -COOR'₄ dans lequel R'₄ représente un atome d'hydrogène ou un groupe alkyle linéaire ou ramifié en C₁-C₄,
un groupe 1H-isoindole-1,3(2H)-dione,
à raison de 1 mole de composé de formule Il pour 1,1 moles de composé de formule R'₃-X, à une température comprise entre 0°C et 200°C et pendant au moins une heure, pour obtenir un composé de formule : 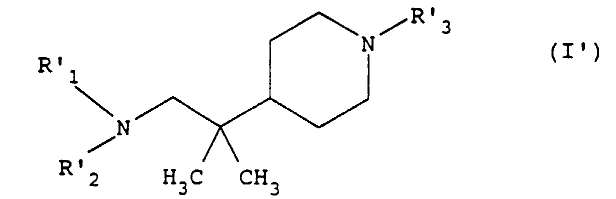 dans laquelle R'₁ , R'₂ et R'₃ sont définis comme ci-dessus;
(ii) si nécessaire, soumettre les composés de formule 1' ainsi obtenus à au moins un des traitements suivants :
(a) les composés de formule 1' dans laquelle R'₃ représente un groupe alkyle linéaire ou ramifié en C₃-C₁₀ comportant une ou plusieurs liaisons C=C ou C≡C et R'₁ et R'₂ sont définis comme précédemment, sont transformés par hydrogénation catalytique dans un alcool en présence d'un catalyseur, en composés de formule I dans laquelle R₁ et R₂, identiques ou différents, représentent chacun un atome d'hydrogène, un groupe alkyle linéaire ou ramifié en C₁-C₄, un groupe phénylméthyle ou forment avec l'atome d'azote auquel ils sont liés un groupe pipéridinyle et R₃ représente une chaîne alkyle en C₃-C₁₀ linéaire ou ramifié;
(b) on réalise une hydrolyse des composés de formule I' dans laquelle R'₁ et R'₂ sont définis comme précédemment et R'₃ représente un groupe alkyle linéaire ou ramifié en C₁-C₄ substitué par un groupe 1H-isoindole-1,3(2H)-dione, en présence d'hydrate d'hydrazine, éventuellement suivie d'une N-alkylation de l'amine primaire ainsi obtenue, par un groupe alkyle en C₁-C₄ pour obtenir les composés de formule I dans laquelle R₁ et R₂, identiques ou différents, représentent chacun un atome d'hydrogène, un groupe alkyle linéaire ou ramifié en C₁-C₄, un groupe phénylméthyle ou forment avec l'atome d'azote auquel ils sont liés un groupe pipéridinyle et R₃ représente un groupe alkyle linéaire ou ramifié en C₁-C₄ substitué par un groupe NR₅R₆ dans lequel R₅ et R₆, identiques ou différents, représentent chacun un atome d'hydrogène ou un groupe alkyle linéaire ou ramifié en C₁-C₄;
c) on réalise une N-acylation de l'amine primaire obtenue au stade (b) précédent par réaction avec un halogénure d'acide de formule X-CO-R₇ dans laquelle X représente un atome d'halogène et R₇ un groupe alkyle linéaire ou ramifié en C₁-C₆, pour obtenir les composés de formule I dans laquelle R₁ et R₂, identiques ou différents, représentent chacun un atome d'hydrogène, un groupe alkyle linéaire ou ramifié en C₁-C₄, un groupe phénylméthyle ou forment avec l'atome d'azote auquel ils sont liés un groupe pipéridinyle, et R₃ représente un groupe alkyle linéaire ou ramifié en C₁-C₄ substitué par un groupe -NH-CO-R₇ dans lequel R₇ représente un groupe alkyle linéaire ou ramifié en C₁-C₆ ;
(d) on amidifie les composés de formule I' dans laquelle R'₁ et R'₂ sont définis comme précédemment et R'₃ représente un groupe alkyle linéaire ou ramifié en C₁-C₄ substitué par un groupe -COOR'₄, dans lequel R'₄ représente un atome d'hydrogène ou un groupe alkyle linéaire ou ramifié en C₁-C₄, par réaction avec une amine primaire, pour obtenir les composés de formule I dans laquelle R₁ et R₂, identiques ou différents, représentent chacun un atome d'hydrogène, un groupe alkyle linéaire ou ramifié en C₁-C₄, un groupe phénylméthyle ou forment avec l'atome d'azote auquel ils sont liés un groupe pipéridinyle et R₃ représente un groupe alkyle linéaire ou ramifié en C₁-C₄ substitué par un groupe -CO-NH-R₇ dans lequel R₇ représente un groupe alkyle linéaire ou ramifié en C₁-C₆;
(e) on réalise la réduction des composés de formule I' dans laquelle R'₁ et R'₂ sont définis comme précédemment et R'₃ représente un groupe -CO-R'₈, dans lequel R'₈ représente un groupe alkyle linéaire ou ramifié en C₈-C₁₁ éventuellement substitué par un groupe imidazolyle, en présence d'un agent réducteur, puis traite avec une base forte pour obtenir les composés de formule I dans laquelle R₁ et R₂, identiques ou différents, représentent chacun un atome d'hydrogène, un groupe alkyle en C₁-C₄, un groupe phénylméthyle ou forment avec l'atome d'azote auquel ils sont liés un groupe pipéridinyle et R₃ représente un groupe alkyle linéaire ou ramifié en C₉-C₁₂ éventuellement substitué par un groupe imidazolyle;
(f) on acyle les composés de formule I' dans laquelle un des deux groupes R'₁ et R'₂ représente l'atome d'hydrogène, l'autre pouvant être un atome d'hydrogène, un groupe alkyle en C₁-C₄ ou un groupe phénylméthyle et R'₃ a la signification donnée à R₃, par réaction avec un anhydride d'acide, pour obtenir les composés de formule I dans laquelle un des groupes R₁ ou R₂ représente un groupe acyle, l'autre pouvant être un atome d'hydrogène, un groupe alkyle en C₁-C₄ ou un groupe phénylméthyle et R₃ représente
un groupe alkyle en C₁-C₁₂ linéaire ou ramifié éventuellement substitué par un groupe imidazolyle ou un groupe phényle lui-même éventuellement substitué par un groupe alkyle en C₁-C₄,
un groupe alkyle linéaire ou ramifié en C₃-C₁₀ comportant une ou plusieurs liaisons double ou triple,
un groupe -Co-R₈ dans lequel R₈ représente un groupe alkyle linéaire ou ramifié en C₈-C₁₁ éventuellement substitué par un groupe imidazolyle,
un groupe alkyle linéaire ou ramifié en C₁-C₄ substitué par :
un groupe -COOR₄ dans lequel R₄ représente un atome d'hydrogène ou un groupe alkyle linéaire ou ramifié en C₁-C₄, un groupe 1H-isoindole-1,3(2H)-dione,
un groupe NR₅R₆ dans lequel R₅ et R₆, identiques ou différents, représentent chacun un atome d'hydrogène ou un groupe alkyle linéaire ou ramifié en C₁-C₄,
un groupe -NH-CO-R₇ ou un groupe -CO-NH-R₇ dans lequel R₇ représente un groupe alkyle linéaire ou ramifié en C₁-C₆.

8. Composé intermédiaire, utile dans la synthèse de composés de formule I selon la revendication 1, caractérisé en ce qu'il s'agit d'un produit de formule 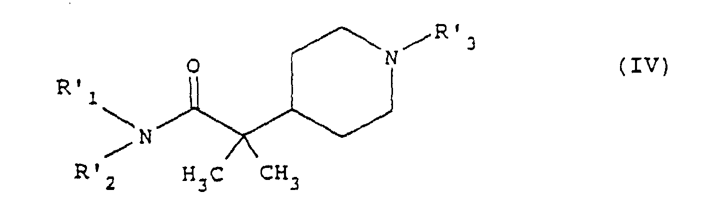 dans laquelle
R'₁ et R'₂, identiques ou différents, représentent chacun un atome d'hydrogène, un groupe alkyle linéaire ou ramifié en C₁-C₄, un groupe phénylméthyle ou forment avec l'atome d'azote auquel ils sont liés un groupe pipéridinyle, et R'₃ représente un groupe alkyle en C₁-C₁₂ linéaire ou ramifié, ou un groupe alkyle linéaire ou ramifié en C₃-C₁₀ comportant une ou plusieurs liaisons C = C ou C≡C.

9. Composition thérapeutique caractérisée en ce qu'elle renferme au moins un composé de formule I ou l'un de ses sels d'addition en quantité thérapeutiquement efficace, en association avec un excipient physiologiquement acceptable.

10. Utilisation d'un composé selon la revendication 1, en tant qu'agent inhibiteur de la biosynthèse du cholestérol, pour l'obtention d'un médicament préventif ou curatif de l'hypercholestérolémie, de l'athérosclérose et des mycoses.

## Patentansprüche

1. Beta,Beta-Dimethyl-4-Piperidinethanamin-Verbindung, dadurch gekennzeichnet, daß sie aus der Gruppe gewählt ist, die durch folgendes gebildet ist:
(i) Verbindungen mit der Formel: 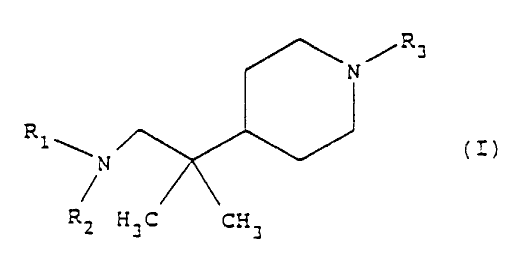 worin
- R₁ und R₂, gleich oder verschieden, jeweils das Wasserstoffatom, eine C₁-C₄-Alkylgruppe, eine C₂-C₅-Acylgruppe, eine Phenylmethylgruppe darstellen oder mit dem Stickstoffatom, an das sie gebunden sind, eine Piperidinylgruppe bilden;
- R₃ folgendes darstellt:
- eine lineare oder verzweigte C₁-C₁₂-Alkylgruppe, die möglicherweise durch folgendes substituiert ist:
a) eine Imidazolylgruppe oder
b) eine Phenylgruppe, die ihrerseits möglicherweise durch eine lineare oder verzweigte C₁-C₄-Alkylgruppe substituiert ist,
- eine lineare oder verzweigte C₃-C₁₀-Alkylgruppe mit einer oder mehreren C=C- oder C≡C-Bindungen, die möglicherweise durch eine Phenylgruppe substituiert ist,
- eine lineare oder verzweigte C₁-C₄-Alkyloxygruppe, die durch eine Parachlorphenylgruppe substituiert ist,
- eine -CO-R₈-Gruppe, in der R₈ eine lineare oder verzweigte C₈-C₁₁-Alkylgruppe, die möglicherweise durch eine Imidazolylgruppe substituiert ist, eine lineare oder verzweigte C₁-C₄-Alkyloxygruppe, die durch eine Parachlorphenylgruppe substituiert ist, oder eine lineare oder verzweigte C₂-C₄-Alkylgruppe darstellt, die wenigstens eine Doppelbindung aufweist und durch eine Phenylgruppe substituiert ist,
- eine lineare oder verzweigte C₁-C₄-Alkylgruppe, die durch eine der folgenden Gruppen substituiert ist:
eine -COOR₄-Gruppe, in der R₄ entweder ein Wasserstoffatom oder eine lineare oder verzweigte C₁-C₄-Alkylgruppe darstellt,
eine 1H-Isoindol-1,3(2H)-dion-Gruppe,
eine NR₅R₆-Gruppe, in der R₅ und R₆, gleich oder verschieden, jeweils ein Wasserstoffatom oder eine lineare oder verzweigte C₁-C₄-Alkylgruppe darstellen,
eine -NH-CO-R₇-Gruppe oder eine -CO-NH-R₇-Gruppe, in denen R₇ eine lineare oder verzweigte C₁-C₆-Alkylgruppe darstellt, sowie
(ii) ihre Additionssalze, insbesondere die Säureadditionssalze und die Ammoniumsalze.

2. Verbindung nach Anspruch 1, dadurch gekennzeichnet, daß das Additionssalz ein ausgehend von einer organischen Säure oder einem Mineral erhaltenes Salz ist.

3. Verbindung mit der Formel I nach Anspruch 1, dadurch gekennzeichnet, daß das Additionssalz ein ausgehend von einem C₁-C₁₄-Alkyliodid erhaltenes Ammoniumsalz ist.

4. Verbindung nach Anspruch 1, dadurch gekennzeichnet, daß es sich um das Salz 1-Dodecyl-N,N,β,β-tetramethyl-4-piperidinethanamin, (E)-2-Butendioat handelt.

5. Verbindung nach Anspruch 1, dadurch gekennzeichnet, daß es sich um das Salz (E)-1-(6,6-Dimethyl-2-hepten-4-in-1-yl)-N,N,β,β-tetramethyl-4-piperidinethanamin, (Z)-2-Butendioat handelt.

6. Verbindung nach Anspruch 1, dadurch gekennzeichnet, daß es sich um das Salz 1-[3-[4-(Methylethyl)phenyl]-2-methyl-propyl] -N,N,β,β-tetramethyl-4-piperidinethanamin, (E)-2-Butendioat handelt.

7. Verfahren zur Herstellung einer Verbindung mit der Formel I oder eines ihrer Salze nach Anspruch 1, das dadurch gekennzeichnet ist, daß es die folgenden Schritte umfaßt:
(i) eine Verbindung mit der Formel 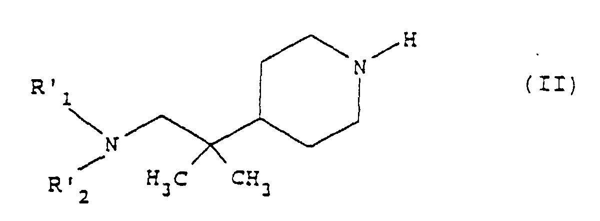 wird einer N-Alkylierungs- oder N-Acylierungsreaktion unterzogen; darin
stellen R'1 und R'₂, gleich oder verschieden, jeweils ein Wasserstoffatom, eine C₁-C₄-Alkylgruppe, eine Phenylmethylgruppe dar oder bilden mit dem Stickstoffatom, an das sie gebunden sind, eine Piperidinylgruppe, und zwar durch Reaktion mit einer Verbindung der Formel R'₃-X, worin X ein Halogen wie beispielsweise ein Brom- oder ein Chloratom und R'₃ folgendes darstellt:
eine lineare oder verzweigte C₁-C₁₂-Alkylgruppe, die möglicherweise durch eine Imidazolylgruppe oder eine Phenylgruppe substituiert ist, die ihrerseits möglicherweise durch eine lineare oder verzweigte C₁-C₄-Alkylgruppe substituiert ist,
eine lineare oder verzweigte C₃-C₁₀-Alkylgruppe mit einer oder mehreren Doppel- oder Dreifachbindungen,
eine -CO-R'₈-Gruppe, in der R'₈ eine lineare oder verzweigte C₈-C₁₁-Alkylgruppe darstellt, die möglicherweise durch eine Imidazolylgruppe substituiert ist,
eine lineare oder verzweigte C₁-C₄-Alkylgruppe, die durch
eine -COOR'4-Gruppe substituiert ist, in der R'₄ ein Wasserstoffatom oder eine lineare oder verzweigte C₁-C₄-Alkylgruppe, eine 1H-Isoindol-1,3(2H)-dion-Gruppe darstellt, im Verhältnis von 1 mol einer Verbindung der Formel II auf 1,1 mol einer Verbindung mit der Formel R'₃-X bei einer Temperatur zwischen 0°C und 200°C und mindestens während einer Stunde, um eine Verbindung mit der folgenden Formel zu erhalten: 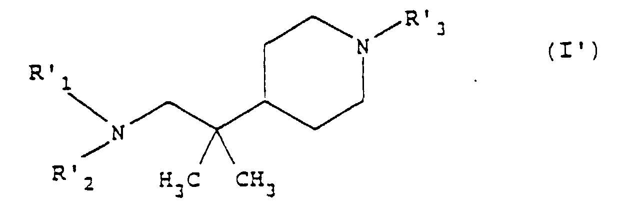 worin R'₁, R'₂ und R'₃ wie oben definiert sind;
(ii) gegebenenfalls werden die derart erhaltenen Verbindungen mit der Formel I' wenigstens einer der folgenden Behandlungen unterzogen:
(a) die Verbindungen mit der Formel I', worin R'₃ eine lineare oder verzweigte C₃-C₁₀-Alkylgruppe mit einer oder mehreren C=C- oder C≡C-Bindungen darstellt und R'₁ und R'₂ wie oben definiert sind, werden durch katalytische Hydrierung in einem Alkohol in Gegenwart eines Katalysators in Verbindungen der Formel I umgewandelt, worin R₁ und R₂, gleich oder verschieden, jeweils ein Wasserstoffatom, eine lineare oder verzweigte C₁-C₄-Alkylgruppe, eine Phenylmethylgruppe darstellen oder mit dem Stickstoffatom, an das sie gebunden sind, eine Piperidinylgruppe bilden und R₃ eine lineare oder verzweigte C₃-C₁₀-Alkylkette darstellt;
b) es wird eine Hydrolyse der Verbindungen mit der Formel I' durchgeführt, worin R'₁ und R'₂ wie oben definiert sind und R'₃ eine lineare oder verzweigte C₁-C₄-Alkylgruppe darstellt, die durch eine lH-Isoindol-1,3(2H)-dion-Gruppe substituiert ist, und zwar in Gegenwart von Hydrazinhydrat, worauf möglicherweise eine N-Alkylierung des derart erhaltenen Primäramins durch eine C₁-C₄-Alkylgruppe folgt, um die Verbindungen mit der Formel I zu erhalten, worin R₁ und R₂, gleich oder verschieden, jeweils ein Wasserstoffatom, eine lineare oder verzweigte C₁-C₄-Alkylgruppe, eine Phenylmethylgruppe darstellen oder mit dem Stickstoffatom, an das sie gebunden sind, eine Piperidinylgruppe bilden und R₃ eine lineare oder verzweigte C₁-C₄-Alkylgruppe darstellt, die durch eine NR₅R₆-Gruppe substituiert ist, worin R₅ und R₆, gleich oder verschieden, jeweils ein Wasserstoffatom oder eine lineare oder verzweigte C₁-C₄-Alkylgruppe darstellen;
c) es wird eine N-Acylierung des im vorhergehenden Schritt b) erhaltenen Primäramins durch Reaktion eines Säurehalogenids mit der Formel X-CO-R₇ durchgeführt, worin X ein Halogenatom und R₇ eine lineare oder verzweigte C₁-C₆-Alkylgruppe darstellt, um die Verbindungen mit der Formel I zu erhalten, worin R₁ und R₂, gleich oder verschieden, jeweils ein Wasserstoffatom, eine lineare oder verzweigte C₁-C₄-Alkylgruppe, eine Phenylmethylgruppe darstellen oder mit dem Stickstoffatom, an das sie gebunden sind, eine Piperidinylgruppe bilden und R3 eine lineare oder verzweigte C₁-C₄-Alkylgruppe darstellt, die durch eine -NH-CO-R₇-Gruppe substituiert ist, worin R₇ eine lineare oder verzweigte C₁-C₆-Alkylgruppe darstellt;
d) man amidiert die Verbindungen mit der Formel I', worin R'₁ und R'₂ wie oben definiert sind und R'₃ eine lineare oder verzweigte C₁-C₄-Alkylgruppe darstellt, die durch eine -COOR'₄-Gruppe substituiert ist, worin R'₄ ein Wasserstoffatom oder eine lineare oder verzweigte C₁-C₄-Alkylgruppe darstellt, und zwar durch Reaktion mit einem Primäramin, um die Verbindungen mit der Formel I zu erhalten, worin R₁ und R₂, gleich oder verschieden, jeweils ein Wasserstoffatom, eine lineare oder verzweigte C₁-C₄-Alkylgruppe, eine Phenylmethylgruppe darstellen oder mit dem Stickstoffatom, an das sie gebunden sind, eine Piperidinylgruppe bilden und R₃ eine lineare oder verzweigte C₁-C₄-Alkylgruppe darstellt, die durch eine -CO-NH-R₇-Gruppe substituiert ist, worin R₇ eine lineare oder verzweigte C₁-C₆-Alkylgruppe darstellt;
(e) es wird die Reduktion der Verbindungen mit der Formel I' durchgeführt, worin R'₁ und R'₂ wie oben definiert sind und R'₃ eine -Co-R'₈-Gruppe darstellt, worin R'₈ eine lineare oder verzweigte C₈-C₁₁-Alkylgruppe darstellt, die möglicherweise durch eine Imidazolylgruppe substituiert ist, und zwar in Gegenwart eines Reduktionsmittels, dann wird eine Behandlung mit einer starken Base durchgeführt, um die Verbindungen mit der Formel I zu erhalten, worin R₁ und R₂, gleich oder verschieden, jeweils ein Wasserstoffatom, eine C₁-C₄-Alkylgruppe, eine Phenylmethylgruppe darstellen oder mit dem Stickstoffatom, an das sie gebunden sind, eine Piperidinylgruppe bilden und R₃ eine lineare oder verzweigte C₉-C₁₂-Alkylgruppe darstellt, die möglicherweise durch eine Imidazolylgruppe substituiert ist;
(f) die Verbindungen mit der Formel I' werden acyliert, worin eine der beiden Gruppen R'₁ und R'₂ das Wasserstofatom darstellt, wobei die andere ein Wasserstoffatom, eine C₁-C₄-Alkylgruppe oder eine Phenylmethylgruppe sein kann und R'₃ die R₃ verliehene Bedeutung hat, und zwar durch Reaktion mit einem Säureanhydrid, um die Verbindungen mit der Formel I zu erhalten, worin eine der Gruppen R₁ und R₂ eine Acylgruppe darstellt, wobei die andere ein Wasserstoffatom, eine C₁-C₄-Alkylgruppe oder eine Phenylmethylgruppe sein kann und R₃
eine lineare oder verzweigte C₁-C₁₂-Alkylgruppe, die möglicherweise durch eine Imidazolylgruppe substituiert ist,
oder eine Phenylgruppe darstellt, die ihrerseits möglicherweise durch eine C₁-C₄-Alkylgruppe substituiert ist,
eine lineare oder verzweigte C₃-C₁₀-Alkylgruppe mit einer oder mehreren Doppel- oder Dreifachbindungen,
eine -CO-R₈-Gruppe, worin R₈ eine lineare oder verzweigte C₈-C₁₁-Alkylgruppe darstellt, die möglicherweise durch eine Imidazolylgruppe substituiert ist,
eine lineare oder verzweigte C₁-C₄-Alkylgruppe, die substituiert ist durch:
eine -COOR₄-Gruppe, worin R₄ ein Wasserstoffatom oder eine lineare oder verzweigte C₁-C₄-Alkylgruppe darstellt, eine 1H-Isoindol-1,3(2H)-dion-Gruppe,
eine NR₅R₆-Gruppe, worin R₅ und R₆, gleich oder verschieden, jeweils ein Wasserstoffatom oder eine lineare oder verzweigte C₁-C₄-Alkylgruppe darstellen,
eine -NH-CO-R₇-Gruppe oder eine -CO-NH-R₇-Gruppe, worin R₇ eine lineare oder verzweigte C₁-C₆-Alkylgruppe darstellt.

8. Zwischenverbindung von Nutzen bei der Synthese von Verbindungen mit der Formel I nach Anspruch 1, dadurch gekennzeichnet, daß es sich um ein Produkt mit der folgenden Formel handelt: 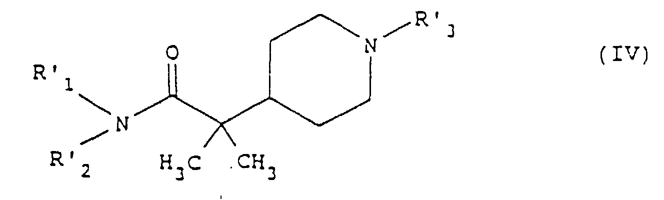 worin R'₁ und R'₂, gleich oder verschieden, jeweils ein Wasserstoffatom, eine lineare oder verzweigte C₁-C₄-Alkylgruppe, eine Phenylmethylgruppe darstellen oder mit dem Stickstoffatom, an das sie gebunden sind, eine Piperidinylgruppe bilden und R'3 eine lineare oder verzweigte C₁-C₁₂-Alkylgruppe oder eine lineare oder verzweigte C₃-C₁₀-Alkylgruppe mit einer oder mehreren C=C- oder C≡C-Bindungen darstellt.

9. Therapeutische Zusammensetzung, dadurch gekennzeichnet, daß sie wenigstens eine Verbindung mit der Formel I oder eines ihrer Additionssalze in therapeutisch wirksamer Menge in Verbindung mit einem physiologisch akzeptablen Arzneimittelträger umschließt.

10. Verwendung einer Verbindung nach Anspruch 1 als Inhibitorwirkstoff für die Cholesterin-Biosynthese zum Erhalt eines präventiven Medikaments oder eines Heilstoffs für Hypercholesterinämie, Atherosklerose und Mykosen.

## Claims

1. A β,β-dimethyl-4-piperidineethanamine compound, characterised in that it is selected from the group consisting of:
(i) compounds of the formula: 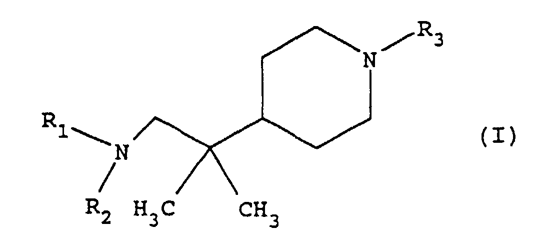 wherein
- R₁ and R₂, which may be identical or different, each represent a hydrogen atom, a C₁-C₄ alkyl group, a C₂-C₅ acyl group or a phenylmethyl group, or form a piperidinyl group with the nitrogen atom to which they are bonded,
- R₃ represents
- a linear or branched C₁-C₁₂ alkyl group which may be substituted by:
a) an imidazolyl group, or
b) a phenyl group which may itself be substituted by a linear or branched C₁-C₄ alkyl group,
- a linear or branched C₃-C₁₀ alkyl group containing one or more C=C or C≡C bonds, and which may be substituted by a phenyl group,
- a linear or branched C₁-C₄ alkyloxy group, substituted by a para- chlorophenyl group,
- a -CO-R₈ group, wherein R₈ represents a linear or branched C₈-C₁₁ alkyl group which may be substituted by an imidazolyl group, a linear or branched C₁-C₄ alkyloxy group, substituted by a parachlorophenyl group, or a linear or branched C₂-C₄ alkyl group containing at least one double bond and substituted by a phenyl group,
- a linear or branched C₁-C₄ alkyl group substituted by one of the following groups:
a -COOR₄ group, wherein R₄ represents either a hydrogen atom or a linear or branched C₁-C₄ alkyl group,
a 1H-isoindole-1,3(2H)-dione group,
a NR₅R₆ group, wherein R₅ and R₆, which may be identical or different, each represent a hydrogen atom or a linear or branched C₁-C₄ alkyl group,
a -NH-CO-R₇ group or a -CO-NH-R₇ group wherein R₇ represents a linear or branched C₁-C₆ alkyl group, and
(ii) their addition salts, in particular their acid addition salts and ammonium salts.

2. A compound according to claim 1, wherein the addition salt is a salt obtained from an organic or inorganic acid.

3. A compound of the formula I according to claim 1, wherein the addition salt is an ammonium salt obtained from a C₁-C₁₄ alkyl iodide.

4. A compound according to claim 1, which is 1-dodecyl-N,N,β,β-tetramethyl-4-piperidineethanamine, (E)-2-butenedioate.

5. A compound according to claim 1, which is (E)-1-(6,6-dimethyl-2-hepten-4-yn-1-yl)-N,N,β,β-tetramethyl-4-piperidineethanamine, (E)-2-butenedioate.

6. A compound according to claim 1, which is 1-[3-[4-(methylethyl)phenyl]-2-methylpropyl]-N, N, β,β-tetramethyl-4-piperidineethanamine, (E)-2-butenedioate.

7. A method for the preparation of a compound of the formula I or one of its salts in accordance with claim 1, said method comprising the steps consisting in :
(i) N-alkylating or N-acylating a compound of the formula: 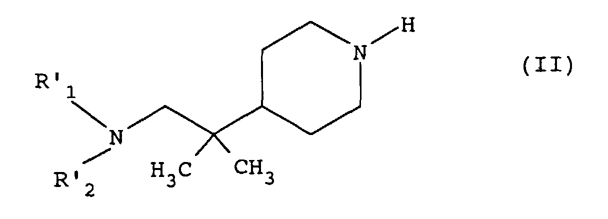 wherein:
R'₁ and R'₂, which may be identical or different, each represent a hydrogen atom, a C₁-C₄ alkyl group or a phenylmethyl group, or form a piperidinyl group with the nitrogen atom to which they are bonded,
by reaction with a compound of the formula R'₃-X wherein X represents a halogen such as a bromine or chlorine atom, and R'₃ represents:
a linear or branched C₁-C₁₂ alkyl group which may be substituted by an imidazolyl group or a phenyl group which may itself be substituted by a linear or branched C₁-C₄ alkyl group,
a linear or branched C₃-C₁₀ alkyl group containing one or more double or triple bonds,
a -CO-R'₈ group, wherein R'₈ represents a linear or branched C₈-C₁₁ alkyl group which may be substituted by an imidazolyl group,
a linear or branched C₁-C₄ alkyl group substituted by
a -COOR'₄ group, wherein R'₄ represents a hydrogen atom or a linear or branched C₁-C₄ alkyl group, or
a 1H-isoindole-1,3(2H)-dione group,
at a molar ratio of 1 mole of compound of the formula II for 1.1 moles of compound of the formula R'₃-X, at a temperature of between 0°C and 200°C for a period of at least one hour, to obtain a compound of the formula: 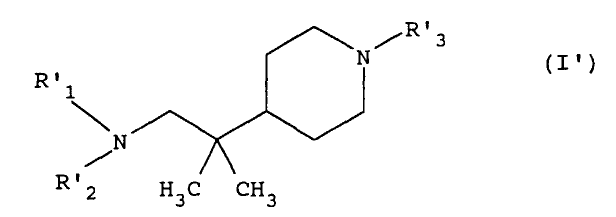 wherein R'₁, R'₂ and R'₃ are as defined above;
(ii) if necessary, carrying out at least one of the following treatments on the compounds of the formula I' thus obtained :
(a) transforming compounds of the formula I' wherein R'₃ represents a linear or branched C₃-C₁₀ alkyl group containing one or more C=C or C≡C bonds and R'₁ and R'₂ are as defined above, by catalytic hydrogenation in a Parr's apparatus, in an alcohol, in the presence of a catalyst, to compounds of the formula I wherein R₁ and R₂, which may be identical or different, each represent a hydrogen atom, a linear or branched C₁-C₄ alkyl group or a phenylmethyl group, or form a piperidinyl group with the nitrogen atom to which they are bonded, and R₃ represents a linear or branched C₃-C₁₀ alkyl chain;
(b) hydrolysing compounds of the formula I' wherein R'₁ and R'₂ are as defined above and R'₃ represents a linear or branched C₁-C₄ alkyl group substituted by a 1H-isoindole-1,3(2H)-dione group, in the presence of hydrazine hydrate, optionally followed by N-alkylation of the primary amine thus produced, using a C₁-C₄ alkyl group to produce compounds of the formula I wherein R₁ and R₂, which may be identical or different, each represent a hydrogen atom, a linear or branched C₁-C₄ alkyl group or a phenylmethyl group, or form a piperidinyl group with the nitrogen atom to which they are bonded, and R₃ represents a linear or branched C₁-C₄ alkyl group substituted by a NR₅R₆ group wherein R₅ and R₆, which may be identical or different, each represent a hydrogen atom or a linear or branched C₁-C₄ alkyl group;
c) N-acylating the primary amine thus obtained in step (b) by reaction with an acid halide of the formula X-CO-R₇ wherein X represents a halogen atom and R₇ represents a linear or branched C₁-C₆ alkyl group, to obtain compounds of the formula I wherein R₁ and R₂, which may be identical or different, each represent a hydrogen atom, a linear or branched C₁-C₄ alkyl group or a phenylmethyl group, or form a piperidinyl group with the nitrogen atom to which they are bonded, and R₃ represents a linear or branched C₁-C₄ alkyl group substituted by a -NH-CO-R₇ group wherein R₇ represents a linear or branched C₁-C₆ alkyl group;
(d) amidifying compounds of the formula I' wherein R'₁ and R'₂ are as defined above and R'₃ represents a linear or branched C₁-C₄ alkyl group substituted by a -COOR'₄ group, wherein R'₄ represents a hydrogen atom or a linear or branched C₁-C₄ alkyl group, by reaction with a primary amine, to obtain compounds of the formula I wherein R₁ and R₂, which may be identical or different, each represent a hydrogen atom, a linear or branched C₁-C₄ alkyl group or a phenylmethyl group, or form a piperidinyl group with the nitrogen atom to which they are bonded, and R₃ represents a linear or branched C₁-C₄ group substituted by a -CO-NH-R₇ group wherein R₇ represents a linear or branched C₁-C₆ alkyl group;
(e) reducing compounds of the formula I' wherein R'₁ and R'₂ are as defined above and R'₃ represents a -CO-R'₈ group, wherein R'₈ represents a linear or branched C₈-C₁₁ alkyl group which may be substituted by an imidazolyl group, in the presence of a reducing agent, then treating with a strong base to obtain compounds of the formula I wherein R₁ and R₂, which may be identical or different, each represent a hydrogen atom, a C₁-C₄alkyl group or a phenylmethyl group, or form a piperidinyl group with the nitrogen atom to which they are bonded, and R₃ represents a linear or branched C₉-C₁₂ alkyl group which may be substituted by an imidazolyl group;
(f) acylating compounds of the formula I' wherein one of the two groups R'₁ and R'₂ represent a hydrogen atom, the other being a hydrogen atom, a C₁-C₄ alkyl group or a phenylmethyl group, and R'₃ has the meaning given for R₃, by reaction with an acid anhydride to obtain compounds of the formula I wherein one of the groups R₁ or R₂ represents an acyl group, the other being a hydrogen atom, a C₁-C₄ alkyl group or a phenylmethyl group, and R₃ represents
a linear or branched C₁-C₁₂ alkyl group which may be substituted by an imidazolyl group or a phenyl group which may itself be substituted by a C₁-C₄ alkyl group,
a linear or branched C₃-C₁₀ alkyl group containing one or more double or triple bonds,
a -CO-R₈ group wherein R₈ represents a linear or branched C₈-C₁₁ alkyl group which may be substituted by an imidazolyl group,
a linear or branched C₁-C₄ alkyl group substituted by:
a -COOR₄ group wherein R₄ represents a hydrogen atom or a linear or branched C₁-C₄ alkyl group,
a 1H-isoindole-1,3(2H)-dione group,
a NR₅R₆ group wherein R₅ and R₆, which may be identical or different, each represent a hydrogen atom or a linear or branched C₁-C₄ alkyl group,
a -NH-CO-R₇ or -CO-NH-R₇ group wherein R₇ represents a linear or branched C₁-C₆ alkyl group.

8. An intermediate compound for use in the synthesis of compounds of the formula I according to claim 1, which is represented by the formula: 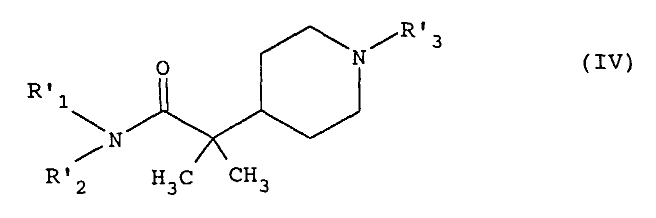 wherein
R'₁ and R'₂, which may be identical or different, each represent a hydrogen atom, a linear or branched C₁-C₄ alkyl group or a phenylmethyl group, or form a piperidinyl group with the nitrogen atom to which they are bonded, and R'₃ represents a linear or branched C₁-C₁₂ alkyl group or a linear or branched C₃-C₁₀ alkyl group containing one or more C=C or C≡C bonds.

9. A therapeutic composition, which comprises at least one compound of the formula I or an addition salt thereof in a therapeutically effective amount, in association with a physiologically acceptable excipient.

10. The use of a compound in accordance with claim 1 as an inhibitor for the biosynthesis of cholesterol to obtain a preventative or curative medicament vis-a-vis hypercholesterolemia, atherosclerosis and mycoses.
